# EUROPEAN PATENT SPECIFICATION

(11) **EP 0 640 130 B1**
(45) Date of publication and mention of the grant of the patent: **15.04.1998**
(21) Application number: 93911146.4
(22) Date of filing: 07.05.1993
(51) Int. Cl.: C12N 15/12, C12N 15/13, C12N 15/62, C12P 21/08, G01N 33/48, G01N 33/534, A61K 38/00, A61K 39/00

(54) **CHIMERIC MULTIVALENT PROTEIN ANALOGUES AND METHODS OF USE THEREOF**
Mehrwertige Chimäre Proteine Anologe und Verfahren zu deren Anwendungen
ANALOGUES DE PROTEINES POLYVALENTS CHIMERES ET PROCEDES D'UTILISATION

(30) Priority: 08.05.1992 US 881109
(43) Date of publication of application: 01.03.1995
(73) Proprietor: CREATIVE BIOMOLECULES, INC., Hopkinton, MA 01748 (US)
(72) Inventor: HUSTON, James, S., Chestnut Hill, MA 02167 (US); KECK, Peter, C., Millbury, MA 01527 (US)
(74) Representative: Holdcroft, James Gerald, Dr.
(86) International application number: US9304338
(87) International publication number: WO9323537

(56) References cited:
- WO-A-88/09344
- PROTEIN ENGINEERING vol. 5, no. 3, April 1992, ENGLAND GB pages 229 - 234 SIMON T;RAJEWSKY K; 'A functional antibody mutant with an insertion in the framework region 3 loop of the VH domain: implications for antibody engineering.'
- BIOCHEMISTRY. vol. 29, no. 6, 13 February 1990, EASTON, PA US pages 1362 - 1367 GLOCKSHUBER R;MALIA M;PFITZINGER I;PLUCKTHUN A; 'A comparison of strategies to stabilize Immunoglobulin Fv-fragments'
- NATURE. vol. 349, 24 January 1991, LONDON GB pages 293 - 299 WINTER, G.; MILSTEIN, C.; 'Man-made antibodies'
- NATURE. vol. 341, 12 October 1989, LONDON GB pages 544 - 546 WARD ES;GUSSOW D;GRIFFITHS AD;JONES PT;WINTER G; 'Binding activities of a repertoire of single immunoglobulin variable domains secreted from Escherichia coli'

## Description

### Background of the Invention

The Immunoglobulin (Ig) Gene Superfamily is comprised of numerous cell surface and soluble molecules that mediate recognition, adhesion or binding functions in vertebrates. (Abbas, A.K. et al., CELLULAR AND MOLECULAR IMMUNOLOGY, p.144 (1991)). Members of the Ig Superfamily have an evolutionary relationship and share significant amino acid sequence and structural similarities. (Williams, A. F. and Barclay, A. N., IMMUNOGLOBULIN GENES, p.372 (1989)). Two criteria for membership within the family are: 1) sequence homology with Ig or Ig-related polypeptide domains, which are approximately 70-110 amino acid residues long, and 2) key structural features which include the polypeptide domains comprised of a sandwich arrangement of two β-sheets, each made up of four or five anti-parallel β-strands of five to ten amino acid residues. (Abbas, A.K., et al., CELLULAR AND MOLECULAR IMMUNOLOGY, pp. 144-145 (1991)).

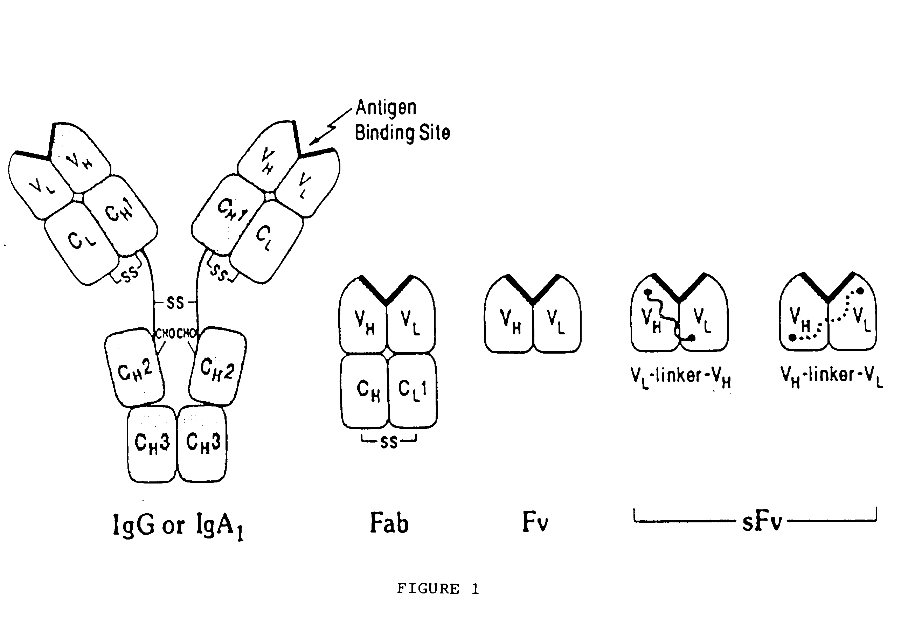

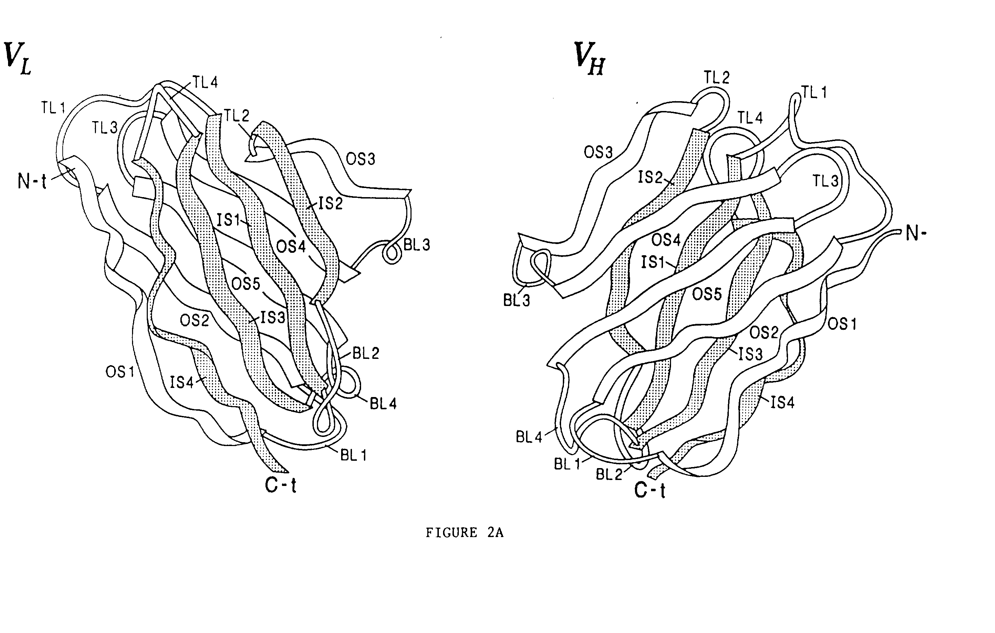

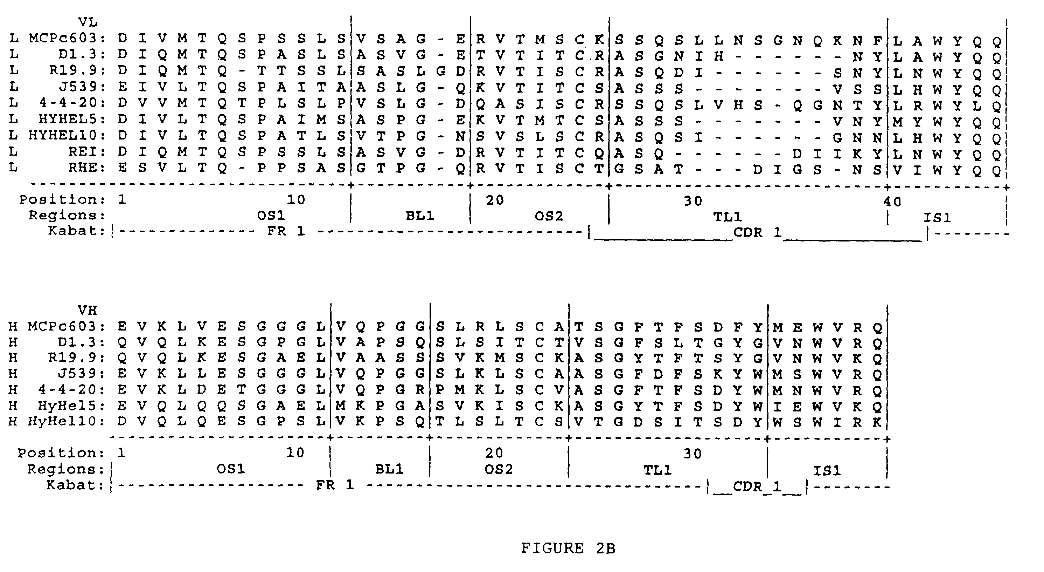

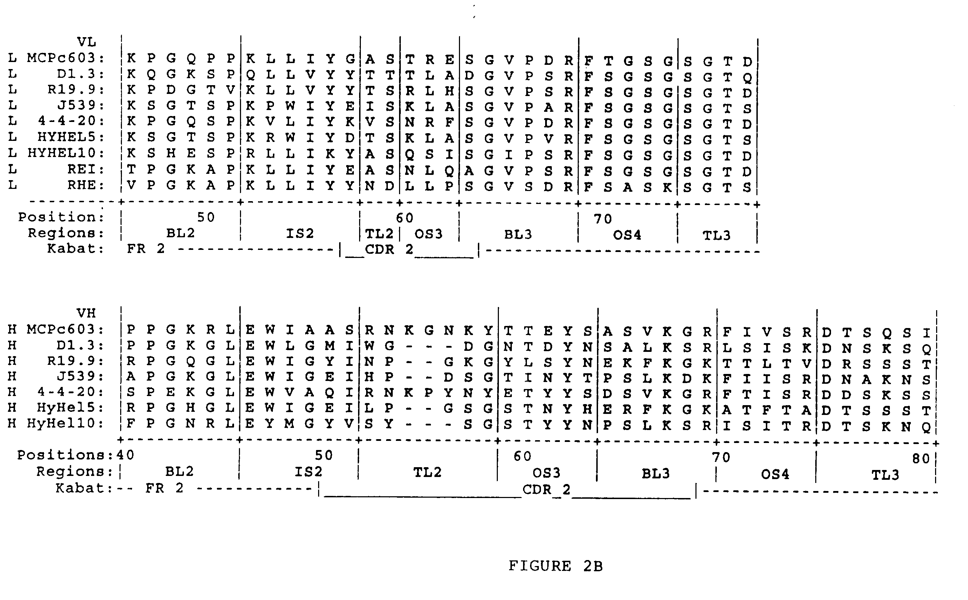

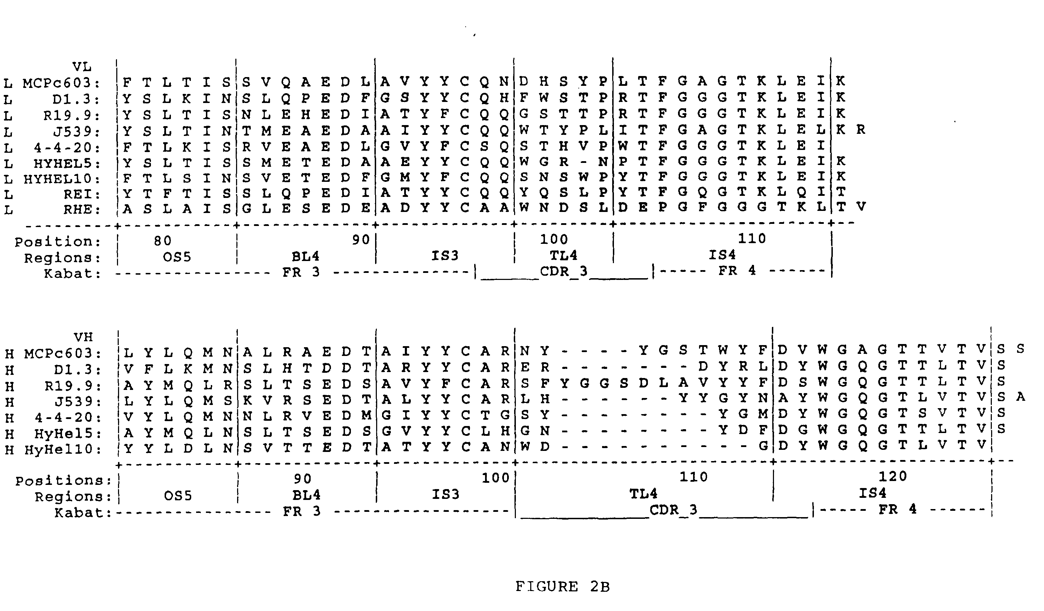

The Ig Superfamily domains are classified as either variable (V) or constant (C) based on characteristics of the β-strands within the β-sheet sandwich. (Abbas, A. K., et al., CELLULAR AND MOLECULAR IMMUNOLOGY, p. 144 (1991)). For example, in one class of Superfamily molecules, the immunoglobulins, V domains are at the amino-terminal ends of separate "heavy" (H) and "light" (L) chains, succeeded in the polypeptide chains by constant (C) domains. Thus, in an immunoglobulin, a V domain is defined as either V_{H} or V_{L} (Figure 1). In the T cell receptor, V and C refer to Ig-like variable and constant domains which are comprised of polypeptide α and β chains. In other Superfamily molecules, V and C domains may be comprised of γ, δ, or ε chains.

In the Ig Superfamily, the polypeptide chains that comprise the V regions, associate to form ligand binding sites. For example, in the immunoglobulin molecule, the V_{H} and V_{L} domains associate to form the variable fragment (Fv) region which comprises the antibody binding site. The Fv region includes both scaffold-like regions, termed framework regions (FRs), and regions of hyper-variability, termed complementarity-determining regions (CDRs). It is the CDRs that contribute to the unique antigen specificity of immunoglobulins. (Abbas, A. K., et al., CELLULAR AND MOLECULAR IMMUNOLOGY, p.45 (1991)). Under special circumstances, the Fv region has been proteolytically dissected from its parent Ig to yield a variable-region fragment (Fv fragment) that is comprised of two non-covalently associated domains (V_{H}·V_{L}, a heterodimer). This heterodimeric Fv fragment can further dissociate into single V_{L} and V_{H} domains. (Huston, J.S. et al. Meth. Enzymol. 203:46-88 (1991)).

Recently, protein engineering methods have been used to link V_{H} and V_{L} chains, creating a functional single-chain Fv (sFv), which has a solitary antibody binding site that does not dissociate into single domains at low concentrations. (Huston, J.S., et al., Proc. Natl. Acad. Sci. USA 85:5879-5883 (August 1988)).

In this approach, the genes encoding V_{H} and V_{L} domains of a given antibody are connected at the DNA level by an appropriate nucleotide sequence, and on translation, this gene forms a single polypeptide chain with a peptide linker bridging the two variable domains. (Huston, J.S., et al., Meth. Enzymol., 203:46-88 (1991)).

### Summary of the Invention

The present invention relates to a chimeric immunoglobulin (Ig) Superfamily protein analogue having more than one biologically active binding site. Hereinafter, the term multivalent will be used to describe these multiple binding sites. The chimeric multivalent Ig Superfamily protein analogue, hereinafter referred to as a CHI-protein, or χ-protein, is comprised of one or more polypeptide chains forming a β-barrel domain. A single β-barrel domain may comprise a chimeric protein binding domain with more than one binding site. Alternatively, more than one β-barrel domain, such as the V_{L} and V_{H} β-barrel domains in immunoglobulins, may combine to form a larger concentric β-barrel domain having more than one binding site.

The β-barrel domain(s) comprising the binding regions has amino acid sequence and structural homology with variable regions of molecules related to the Ig Superfamily of molecules. Specifically, the binding sites on the χ-protein are comprised of hypervariable regions derived from molecules related to the Ig Superfamily of molecules. The Ig Superfamily includes immunoglobulins, cell surface antigens, such as T lymphocyte antigens, and cell surface receptors, such as immunoglobulin Fc receptors.

In a preferred embodiment, the hypervariable regions are complementarity-determining regions (CDRs) derived from the antigen binding sites of immunoglobulins. In this embodiment, the multivalent protein analogue comprises one or more polypeptide chains forming a β-barrel domain containing CDRs interspersed between framework regions (FRs). These CDRs define one antigen binding site. This multivalent protein analogue also has one or more additional antigen binding sites spliced into the FRs of the β-barrel domain.

In one embodiment, the χ-protein will comprise a single polypeptide chain forming a β-barrel domain. In another embodiment the χ-protein will comprise a single polypeptide chain comprised of two polypeptide chains, connected by a polypeptide linker spanning the distance between the C-terminus of one chain to the N-terminus of the other chain forming a β-barrel domain. In yet another embodiment, the χ-protein will comprise two polypeptide chains with two non-covalently associated chains forming a β-barrel domain. In each of the above embodiments, the polypeptide chains fold to form a β-barrel domain with two or more binding sites.

The invention also relates to the amino acid sequences that encode the χ-protein, the DNA sequences that encode the amino acid residues forming the χ-protein, and expression vectors comprising and capable of expressing the DNA sequences. The invention also relates to methods of producing the χ-Protein.

The invention further relates to compositions comprised of the χ-proteins and methods of use thereof. These compositions are comprised of χ-proteins having two biologically active binding sites and may be used in a variety of therapeutic and diagnostic procedures. These compositions include, but are not limited to, χ-protein biosensors which undergo a conformational change when a ligand is bound to one binding site such that the affinity of the second binding site is modified; and χ-proteins having one binding site reactive with a tissue-specific ligand and the second binding site reactive with radioactive ions, radio-opaque substances, cytotoxic substances, cytotoxic effector cells (e.g. cytotoxic T cells) drugs, or catalytic substances. These compositions also include a "biochip" comprised of a two-dimensional array of aggregated χ-protein biosensors, such as in a Langmuir-Blodgett film, to make a functionalized membrane useful for layers of molecular gates for computers and the like.

The utility of binding proteins having two independent binding sites of different specificity for the treatment or control of tumors, virus infected cells, bacteria and other pathogenic states has been recognized (Segal, D.M. and Snider, D.P., Chem. Immunol. 47:179-213 (1989)). Bispecific binding proteins have been produced by crosslinking two or more dissimilar but intact antibodies with a chemical agent (heteroantibodies); crosslinking antibody fragments; linking two single chain antibodies; or fusing a single chain antibody to an effector molecule (Segal et al. U.S. 4,676,980), Tai, M., et al. Biochem. 29:8024-8030 (1990).

However, despite considerable application to medical research, these previous attempts to produce bispecific binding proteins suffer from the difficulty of complex purifications and large molecular size. For example, each binding region of an IgG is at least 50 kD, so that a conventional crosslinked, bispecific heteroantibody can range in mass from 100-150 kD to as much as 300 kD, if two intact IgG antibodies are crosslinked. Even the single chain antibody has a mass of approximately 26 kD so that a single polypeptide chain comprising two separate binding regions, each comprised of a single chain antibody, has a mass of approximately 50 kD.

The recombinant-engineered χ-proteins of the present invention have significant advantages over conventionally crosslinked antibodies, or even the single-chain Fv. The proteins of the present invention can be custom-designed to bind specific ligands and cell surface receptors with affinity or specificity. These custom-designed multivalent binding proteins can be smaller and more compact in size than intact hetero-bispecific antibodies, Fab' antibody binding fragments or bispecific sFv-sFv constructs.

These χ-proteins can also be less immunogenic thereby reducing the likelihood of immune reactions to such therapeutic compositions. For example, in the intact immunoglobulin molecule, the bottom loops of the variable region are sterically protected from recognition resulting in an immune response. However, when disassociated from the protecting constant region, as in the Fv, the exposed bottom loop region potentially becomes antigenic. In a χ-protein, this bottom loop region is no longer exposed and thus significantly reduces the likelihood of an immune response. Furthermore, due to their smaller size, the χ-proteins can have enhanced stability when administered intravenously as they will be less susceptible to proteolysis by endogenous proteases than larger, multi-domain proteins.

### Brief Description of the Drawings

The foregoing features and advantages of the invention will be apparent from the following more particular description in the following drawings and text.

Figure 1 is a schematic representation of a typical Ig Superfamily molecule, an immunoglobulin, depicting the variable (V) and constant (C) regions.

Figure 2A is a schematic representation of an Fv depicting the relative positions of the top loops (TL) and bottom loops (BL) of the folded heavy and light chains. The TLs typically form the CDRs of the Fv and the BLs are typically adjacent to the C region when within an intact immunoglobulin. The BLs comprise the loops suitable for splicing on the second binding site. The shaded strands are the inner strands (IS). The unshaded strands are the outer strands (OS).

Figure 2B shows an alignment of V_{L} and V_{H} amino acid sequences for which three dimensional structures are known (SEQ ID NOS: 1-9 for V_{L} and SEQ ID NOS 10-16 for V_{H}). The alignment of these sequences is based on the structural homology that exists between them, especially in the β-strand regions. Regions of the sequence corresponding to structural regions of inner-β-strand (IS), outer-β-strand (OS), top loops (TL) and bottom loops (BL) are identified. The number scale corresponds to structural position, especially in the OS and IS regions.

Figure 3A is a stereo figure depicting two copies of the McPC 603 Fv structure wherein the top loops of one (right side up) structure are superimposed with the bottom loops of the other (upside down) structure. For clarity, only the top loops (ribbons) and bottom loops (line trace) are shown.

Figure 3B is a stereo figure depicting the alignment of H2 with the N- and C-terminal strands of the inverted Fv structure.

Figures 4A-4G depict in stereo the positions of the native CDRs and the CDRs spliced into the McPC 603 bottom loops to form a additional binding site (χ-site). Corresponding native and χ-site CDR loops are highlighted as ribbons.

Figure 5 depicts the stereo comparison of the χ-protein comprised of native McPC603 with McPC603 CDRs spliced into the BLs of the native McPC603.

Figure 6 depicts the splice points used when H3 of a second McPC603 is spliced into H BL2 of the native McPC603 sFv. The ribbon follows the spliced trace.

Figure 7 depicts the splice points used when the L3 loop is spliced into the L LB2 of the native McPC603.

Figure 8 depicts the splice points used when the H1 loop is spliced into H BL4 of the native McPC603.

Figure 9 depicts the splice points used when the L1 loop is spliced into L BL4 of the native McPC603.

Figure 10 depicts the splicing of the H2 loop into the C-terminus of V_{H} of native McPC603.

Figure 11 depicts the splicing of the L2' loop onto the C-terminus of V_{L} of native McPC603.

Figure 12 shows the final amino acid sequence of the χ-protein comprised of two McPC603 binding sites as constructed according to Examples 1 and 2 (SEQ ID NO: 17).

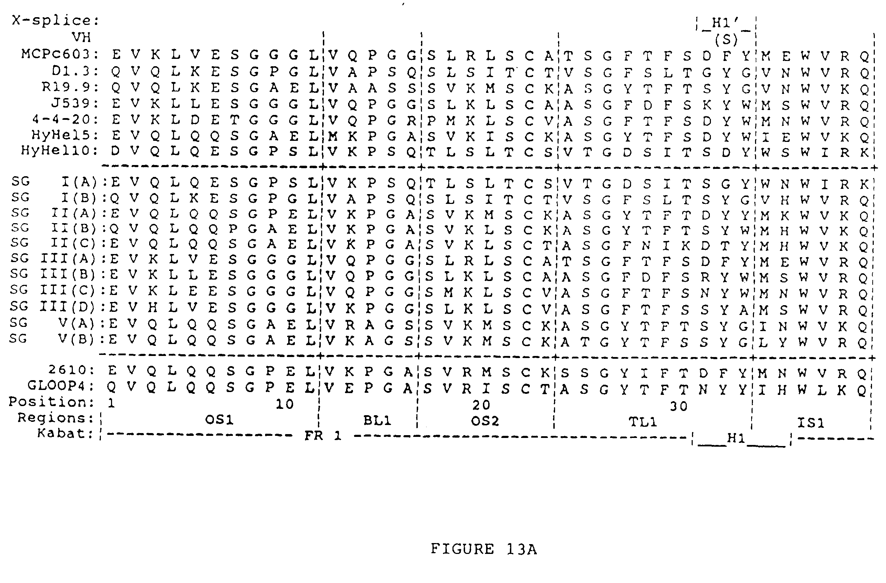

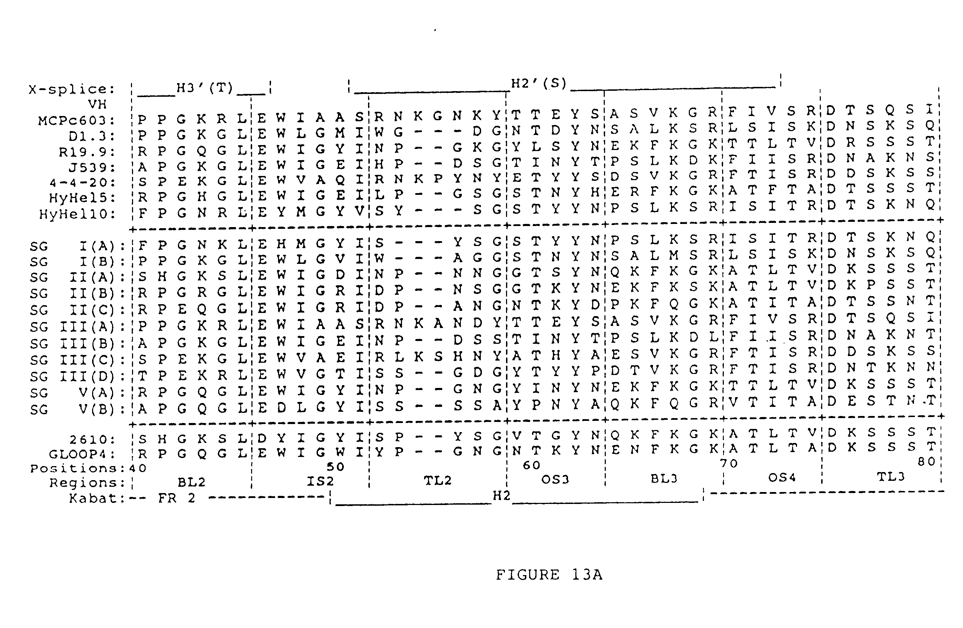

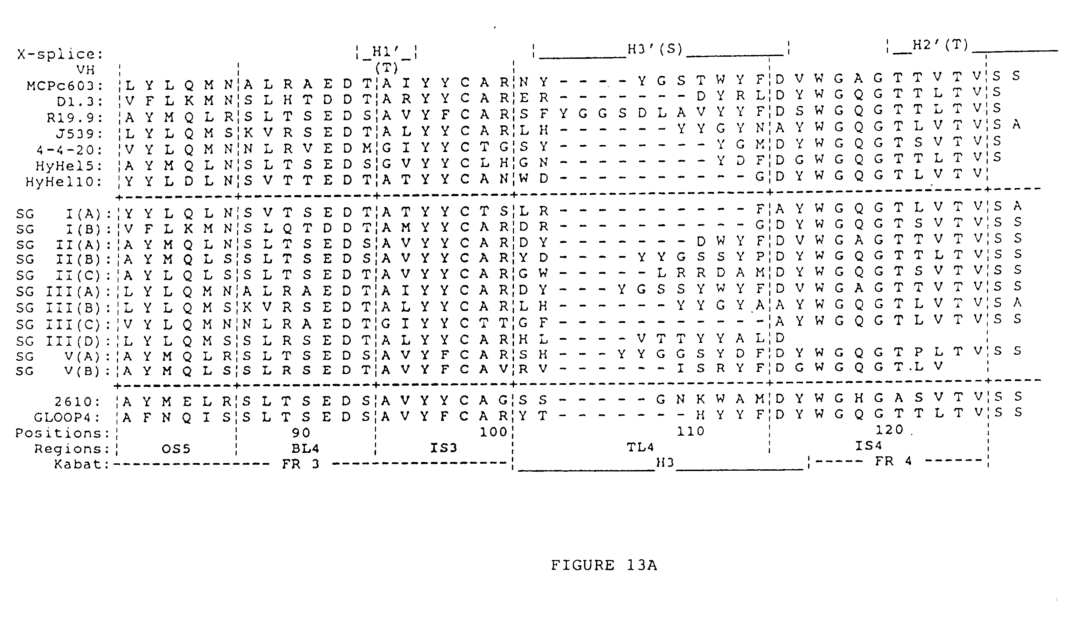

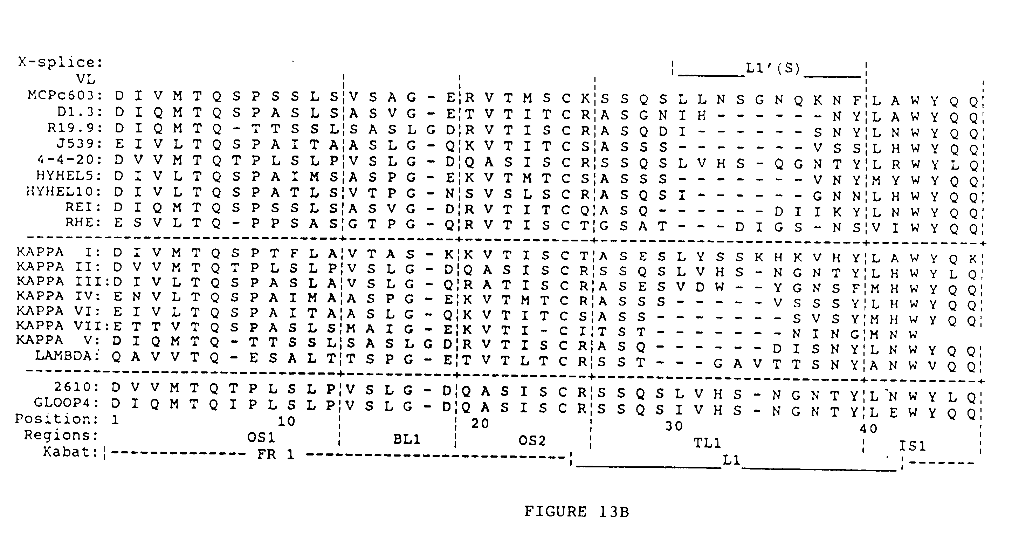

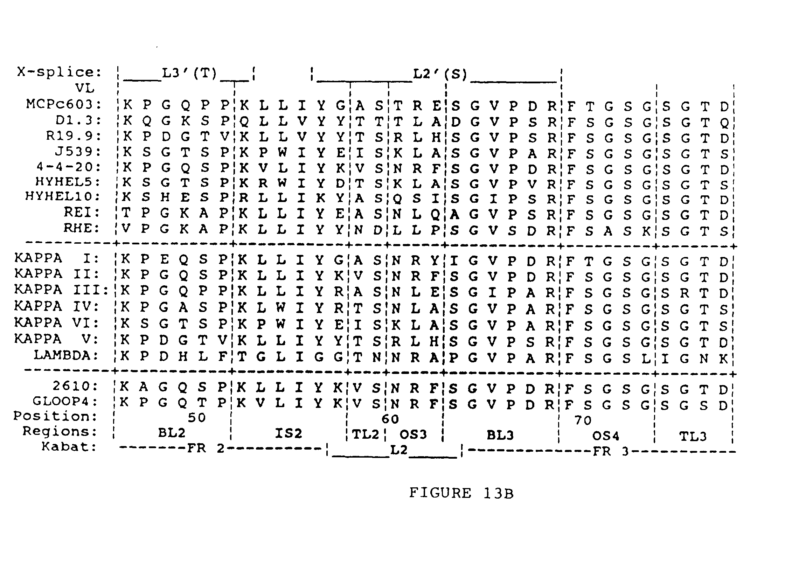

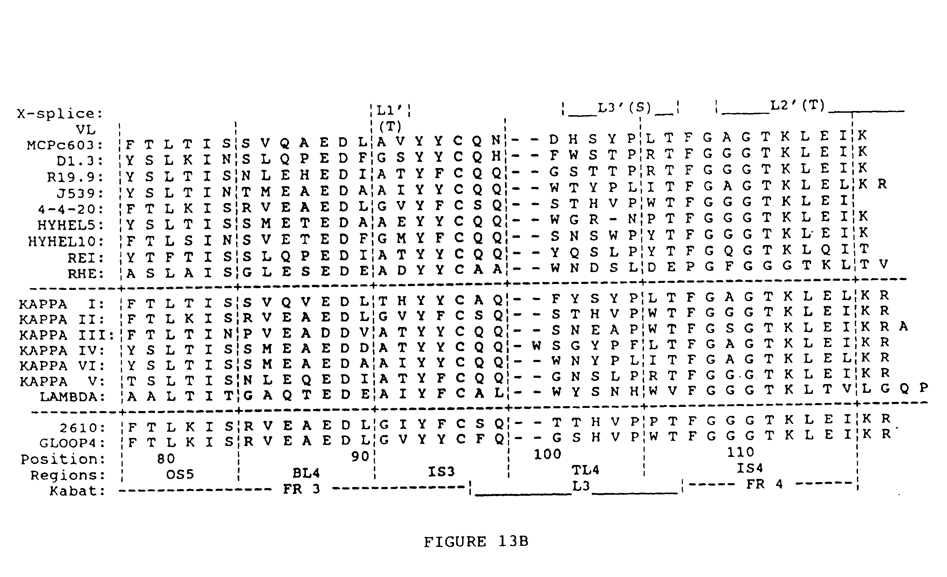

Figures 13 A and B show the alignment of consensus sequences for the various sequence classes in the Kabat et al. compendium with the alignment of sequences of known structure for the V_{H} (Figure 13A) and V_{L} (Figure 13B) chains; (Kabat, E.A., SEQUENCES OF PROTEINS OF IMMUNOLOGICAL INTEREST Vols. I-III, U.S. Dept. Health and Human Services, NIH Pub. No. 91-3242 (1991)). The residue preference list in Table 2 is derived from this figure. The first group of sequences are as in Figure 2B (SEQ ID NOS: 1-16). The second group of sequences are consensus sequences from the groupings in Kabat et al. (Figure 13A, SEQ ID NOS: 18-28) and Figure 13B, SEQ ID NOS: 31-38). The third group of sequences are known sequences for mouse immunoglobulins 2610 and GLOOP4. (Figure 13A, SEQ ID NOS: 29-30 and Figure 13B, SEQ ID NOS: 39-40). The line labeled KABAT indicates the boundaries of framework and CDR regions as defined in Kabat et al. ("-" indicate alignment gaps).

Figure 14 shows the sequences of χ-protein constructs as constructed according to Examples 2 and 3 (SEQ ID NOS: 41-44).

Figure 15A shows the nucleic acid and amino acid sequences of the χ-1 protein, with double dashes indicating the D1.3H1 χ-loop insertion in the 26-10 sFv (SEQ ID NOS: 45 AND 46, respectively).

Figure 15B shows the nucleic acid and amino acid sequences of the χ-2 protein, with double dashes indicating the D1.3H1 and H2 χ-loop insertions in the 26-10 sFv (SEQ ID NOS: 47 AND 48, respectively).

Figure 16A shows the SDS polyacrylamide gel electrophoresis of the χ-1 protein purified by ouabain-Sepharose affinity chromatograph.

Figure 16B shows the SDS polyacrylamide gel electrophoresis of the χ-2 protein purified by ouabain-Sepharose affinity chromatography.

Figure 17 shows the alution profiles of the χ-2 protein and the 26-10 sFv from a Superdex 75 column.

### Detailed Description of the Invention

The present invention relates to a chimeric multivalent immunoglobulin (Ig) Superfamily protein analogue, hereinafter referred to as a χ-Protein, comprising one or more polypeptide chains forming a β-barrel domain. The β-barrel domain, contains hypervariable regions (hereinafter called complementarity-determining region-like (CDR-like region)) and structural regions (hereinafter called framework-region-like (FR-like region)). The CDR-like regions define ligand binding sites. Additionally, the χ-protein has at least one more ligand binding site segment spliced into the FR-like regions of the β-barrel domain.

The Ig Superfamily molecules show significant amino acid sequence homology within their β-barrel domains. (Williams, A.F. and Barclay, A.N., IMMUNOGLOBULIN GENES p. 362 (1989)). The amino acid sequences of the amino terminal domains are called variable (V) regions and the more conserved sequences of the remainder of the chain, termed the constant (C) region. (Figure 1) (Abbas, A. K., et al., CELLULAR AND MOLECULAR IMMUNOLOGY p. 45 (1991)). The amino acid sequences of both the V and C regions of Superfamily molecules are formed on two different polypeptide chains, the heavy (H) chain and the light (L) chain in immunoglobulins, or the α, β, γ, δ, or ε chains in other Ig Superfamily molecules. These chains also fold into V and C regions. For example, each H chain of an immunoglobulin molecule folds into a V_{H} domain with an adjacent C_{H} domain and each L chain folds into a V_{L} domain with an adjacent C_{L}. Each chain has additional successive constant regions (Figure 1).

The V region contains highly variable, unconserved stretches of sequence called the hypervariable regions. More conserved stretches of sequence are called structural regions. In immunoglobulins, the hypervariable regions are called complementarity-determining regions (CDRs) and the structural regions are called framework regions (FRs). Three CDRs of each V_{H}, and three CDRs of each V_{L} combine in a unique three-dimensional structure to form the antigen or ligand binding site. These CDRs determine the ligand specificity of the protein. (Abbas, A. K., et al., CELLULAR AND MOLECULAR IMMUNOLOGY p.143 (1991)).
Hereinafter, the hypervariable regions of all Ig Superfamily molecules will be called CDR-like and all conserved regions will be called FR-like (or CDRs and FRs in the specific case of an immunoglobulin molecule).

The Ig Superfamily members also show significant homology in the structural three-dimensional features of their V and C domains. This structural feature is known as the Ig-fold. (Williams, A.F. and Barclay, A.N., IMMUNOGLOBULIN GENES p. 362 (1989)). The Ig-fold consists of a sandwich of two β-sheets constructed from anti-parallel β-strands, each strand containing five to ten amino acid residues. The V domain differs from the C domain by an extra pair of β-strands in the middle of the V domain. (Williams, A.F. and Barclay, A.N., IMMUNOGLOBULIN GENES p. 362 (1989)). For example, the V domains or C domains of an immunoglobulin, associate in pairs, such as V_{H°}V_{L}. Each β-barrel domain, when associated in such a pair, forms two concentric β-barrels, with the CDR loops connecting anti-parallel β-strands of the inner barrel.

Members of the Ig Superfamily include, but are not limited to, Immunoglobulins, T cell Receptor Complex, Major Histocompatibility Complex Antigens, β₂ Microglobulin-associated Antigens, T Lymphocyte Antigens, Growth Factor Receptors, and Neural Cell Adhesion Molecules (NCAM). (Williams, A.F. and Barclay, A.N., IMMUNOGLOBULIN GENES p. 362 (1989)).

Each ligand binding site of the χ-protein is comprised of the CDR-like region derived from molecules of the Ig superfamily. For example, a ligand binding site could be comprised of the CDRs derived from an immunoglobulin molecule whose ligand is an antigen. Alternatively, the ligand binding site could be comprised of CDR-like regions from a receptor molecule such as the T cell receptor whose ligand is the antigen-major histocompatibility complex (MHC) molecule which, upon binding to its receptor, initiates T cell activation.

In particular, the present invention relates to the immunoglobulins of the Ig Superfamily. In natural immunoglobulins, the antibody combining site is formed by CDRs of the V_{H} and V_{L} variable domains within the Fv (variable region consisting of noncovalently associated V_{H} and V_{L} or V_{H°}V_{L}), as shown in Figure 1. In addition to the CDRs determining binding specificity, maintenance of the tertiary structure is also necessary for biological activity. The CDRs are correctly positioned by the conserved framework regions (FRs) within the V regions, and the V region is further stabilized by the C regions of the protein. However, the minimal naturally-occurring antibody binding site is the two chain, non-covalently associated Fv.

Recently, through recombinant protein engineering, a single-chain Fv (sFv) has been constructed. In this approach, the genes encoding V_{H} and V_{L} domains of a given antibody are connected at the DNA level by an appropriate oligonucleotide linker and, on translation, this gene forms a single polypeptide chain with a peptide linker bridging the two variable domains. (Huston, J.S. et al., Meth. Enzymol. 203:46-48 (1991)).

Isolated single domain antibodies have also recently been constructed, comprised of only three CDRs instead of the usual complement of six. (Ward, E. S., et al., Nature 341: 544-546 (1989)). In some cases, these single domain antibodies (i.e., V_{H} or V_{L}) exhibit binding activities comparable to their parent antibodies.

In a preferred embodiment of the present invention, one of the χ-Protein binding sites is comprised of a set of CDRs from a mouse myeloma protein such as 26-10 or McPC603 (Huston, J.S., et al. Methods Enzymol. 203: 46-88 (1991). An additional binding site segment is spliced into the β-barrel domain. This spliced segment is comprised of CDRs from the same, or a second mouse myeloma protein, which are spliced into the bottom FR loops of the β-barrel domain, or attached to the C-terminal ends of each V domain.

The structural basis for this invention can be explained with reference to Figure 2A and 2B (SEQ ID NOS: 1-16), where the typical variable region composition of an immunoglobulin is noted. There are three CDRs within each V_{H} and V_{L} which together constitute a complete antibody binding site. These CDRs are interspersed between FRs such that the light chain V region is denoted FR1-L1-FR2-L2-FR3-L3-FR4 and the heavy chain is denoted FR1-H1-FR2-H2-FR3-H3-FR4. Each of these V region chains folds into a native conformation that comprises a double layer of β-sheets. V domains may be monomeric, (V_{H} or V_{L}), or associate into homodimers, (V_{H°}V_{H} or V_{L°}V_{L}), or into the heterodimeric Fv, (V_{H°}V_{L}). Each of these dimers may be constructed as single-chain analogues. In the single-chain Fv, these two sequences are connected in tandem with a bridging linker to make, for example, V_{H}-linker-V_{L} (V_{H}-V_{L}) or V_{L}-linker-V_{H} (V_{L}-V_{H}).

In these folded configurations of the V region polypeptide, alternating directions of the β-strands loop out at either end as they form anti-parallel strands. In each region the immunoglobulin fold has loops on top (the binding site is "on top") and on the bottom (partly in close proximity to constant domains for intact H and L chains).

On top of each V domain, four loops are present of which three are CDRs that contribute to the antigen binding site, and on the bottom, four loops are present that allow the β-strands to switch directions and fold back into the globular domain. These bottom loops are here termed BL1, BL2, BL3, and BL4, and apply to the L and H variable regions, respectively called L BL1-4 and H BL1-4 (Figure 2A). These bottom loops provide insertion sites for fusion proteins or peptide segments, which are further augmented by splicing of peptide sequence at the C-terminus of each V region. The use of these bottom loops as splice sites permits incorporation of alternate binding, catalytic or effector sites which complement the naturally present antigen binding site.

However, novel demands are made on variable region architecture in order to construct additional binding sites on the bottom of a V domain. In particular, there is a requirement that the relative orientation of the CDR-like loops (CDR or top loop symmetry) for an Fv region be reproduced to a reasonable approximation in the relative orientation of the bottom loops of the Fv (bottom loop symmetry). Whether or not this symmetry relationship exists is not an obvious question to ask and the possibility of top-bottom loop symmetry has not been previously recognized. Nonetheless, molecular modeling and computational analysis demonstrate that such an approximation to the CDR-like loop symmetry does exist for the bottom loops of the Fv region.

The superposition of top and bottom loops involves two interrelated observations: (1) the two fixed endpoints of each top loop (CDR) must find a corresponding match with bottom loop endpoints; and (2) the polypeptide chain directionality must be maintained after bottom loop splicing (i.e., N to C directionality of the spliced CDR must be the same as the bottom loop that it replaces). The overall architecture of the Fv region appears asymmetric because the ends of the light and heavy chain β-barrels are closer together at the top than they are at the bottom. However, much of this apparent difference derives from there being one quarter of the Fv residues in CDRs on top of the framework, which fill in space that is open on the bottom.

In fact, there is sufficient correlation between top and bottom loop symmetry of the intact Fv region to make the multiple binding site molecule possible. This is discernible if one superimposes the top of one Fv (#1) with the bottom of another Fv (#2); if #1 and #2 are the same, then the correlation helps decide how to construct a multivalent Fv, whereas if #1 and #2 are different, the additional binding site (χ-site) is distinct from the native Fv binding site, and overall one thereby designs a chimeric multivalent Ig Superfamily protein analogue which has one or more sites of different specificity.

We will represent the composition of the χ-site with parentheses so that A(B) represents a χ-protein comprising the CDR-like regions of immunoglobulin Superfamily molecule B built into the χ-site of immunoglobulin Superfamily molecule or analogue A. Likewise (V_{H}(V_{L}) represents a V_{H} domain with a χ-site whose loops are derived from the CDR loops of a V_{L} domain. Consequently the designation A(B) V_{H}(V_{L})-V_{L}(V_{H}) denotes a χ-protein based on the FR and CDR of Fv A having a χ-site based on the CDR loops of Fv B where the χ-site loops on the heavy chain of A are derived from the CDR loops of the light chain B and *visa versa,* and the two χ domains are linked by a polypeptide linker such that the heavy chain FR domain precedes the light chain FR domain.

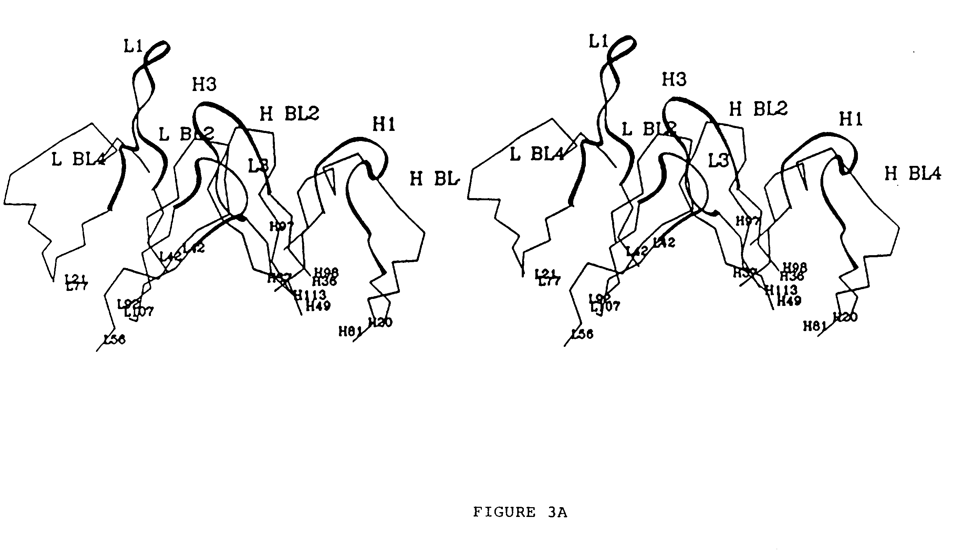

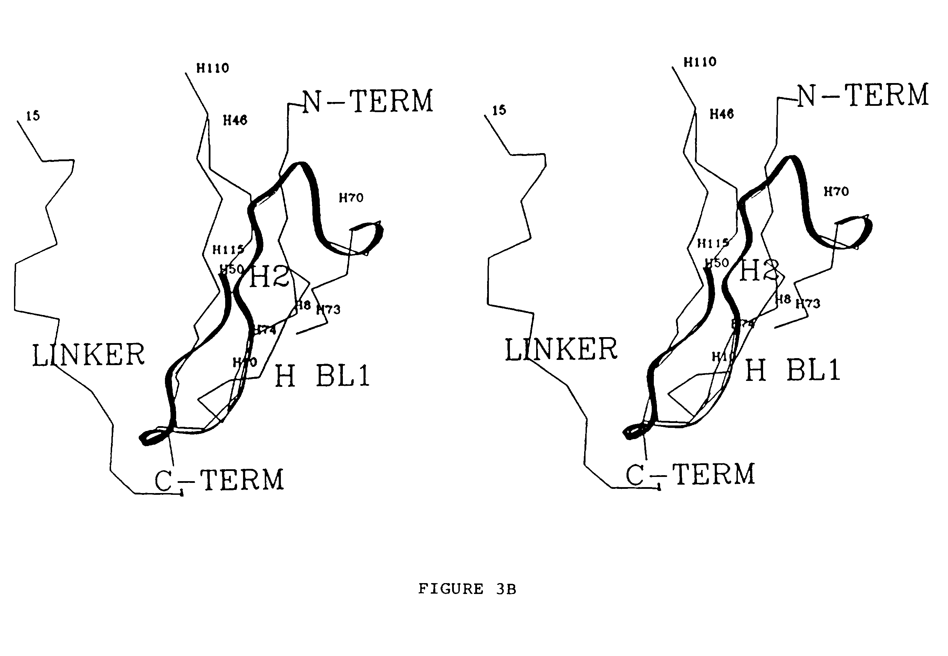

As shown in Figure 3A, by applying these procedures to McPC603 for both Fv #1 and #2, the following pairs of loops align:
1. The TL4 loops (L3 and H3) of Fv#1 can be aligned very closely with the BL2 loops of Fv#2, and with the alignment of these two sets of loops, additional alignments become apparent.
2. The TL1 loops (L1 and H1) are approximately superimposed on the respective BL4 loops.
3. In addition, as shown in Figure 3B, the ends of the TL2 loops (L2 and H2) are approximately superimposed on the N-terminal and C-terminal strands of the respective β-barrel domains.

In this structural alignment, all of the fundamental design criteria for splicing a second binding site onto the bottom of V, Fv, or sFv regions are satisfied: the proper chain directionalities and superpositions are found for the TL4 - BL2 pair, the TL1 - BL4 pair, and the ends of the TL2 loops relative to the N- and C-terminal strands of the β-barrel region.

This construction is consistent to a good approximation with the natural geometry for all CDR loops, thereby generating a ligand binding site on the bottom of the β-barrel domain similar to the "source" Fv binding site. Similarly, an additional ligand binding site may be built on the separate non-covalently linked V domains of a heterodimeric Fv (i.e., V_{H°}V_{L}). Alternatively, as it has recently been shown that the full set of 6 CDRs is not necessary for ligand binding, partial binding sites (i.e., fewer than 6 CDRs) may be assembled on a single V domain. Thus, a χ-protein could comprise a single β-barrel domain with two ligand binding sites, each binding site with as few as one CDR and still exhibit binding activity.

H BL1 or L BL1 loop replacement could also be useful, as they are in proximity to the rest of the binding site. However, their peptide chain directionality is opposite to what would be needed to correctly splice in H2 and L2 loops. Nonetheless, such loops could be devised de novo by design or mutagenesis to facilitate such replacement.

Furthermore, in most Fv regions, the H or L BL3 loops are located on the sides of the V domains.
Although these loops are not contiguous with the rest of the additional binding site, ancillary loops could be attached at such sites, thereby providing the addition of a "ligand-like" surface feature for recognition by the appropriate receptor, thus, forming a χ-protein with more than two binding sites. A single substitution of only one loop, with an appropriate peptide, could provide a means to anchor the binding protein to a particular receptor. Moreover, the H or L BL3 (or H or L TL3) loops could provide the means to crosslink single domain χ-proteins into aggregate sheets to form two dimensional arrays of χ-proteins.

CDR-like region sequences of any of the proteins from the Ig Superfamily showing the requisite sequence and structural homology can be spliced into the bottom loops of the source Fv. It should be noted that CDR-like region sequences from other Ig Superfamily molecules can also replace all or a part of the native CDR-like region of the χ-protein. Thus a χ-protein can comprise the CDR-like regions from molecule A, the FR-like regions from molecule B and the χ-site having CDR-like regions from molecule C spliced in.

As discussed in detail in Examples 1 and 2, using the Fv derived from mouse myeloma IgA antibody, McPC 603, a second McPC 603 binding site can be constructed on the bottom of the source Fv. Although the Fv region of McPC 603 is exemplified, as shown in Example 3, it is reasonable to splice in a CDR-like region from other Ig Superfamily proteins, due to the sequence and structural homology that exists among these proteins. (Figure 2B (SEQ ID NOS: 1-16)).

When CDR-like sequences are spliced into the lower FR-like loops of the source Fv, it is important to preserve those FR-like residues that are critical for maintaining the proper folding. These critical FR-like residues are located in the stems of the loops and underlying the CDR-like regions. Three criteria govern the selection of the proper splice points. First, as discussed above, the need to preserve critical FR-like residues; second, the desire to incorporate as much of the CDR-like sequence as possible; and third, the practical need to switch from one backbone to the other at points where the alpha carbons of the two chains are reasonably well aligned. The last requirement is necessary in order for the spliced loops to maintain their native, biologically active conformation.

As described in detail in Examples 1 and 2, the crystallographically determined coordinates of a mouse myeloma protein structure, such as McPC603, are visually displayed using a suitable computer graphics system. This display of the protein in three-dimensions, can be spatially rotated, turned or twisted as necessary to view the polypeptide chains or peptide backbone of the Fv region. Using the graphics program, substitutions, additions or deletions can be made to the structure. These modifications are energy minimized (optimized) to account for steric hindrance, bond lengths, bond angles and energy constraints so as to maintain the critical tertiary structure necessary for biological binding activity. Thus, in a step by step process involving modification of the polypeptide chain and successive cycles of refinement calculations for each modification, a three-dimensional model of the χ-protein, with two distinct binding sites, is built.

As described in Examples 1 and 2, the model used was McPC603 sFv constructed as V_{H}-V_{L}. However, sFv proteins constructed as V_{L}-V_{H}, V_{H}-V_{H}, or V_{L}-V_{L} are also intended to be encompassed by this invention. Two chain Fv regions (i.e., non-covalently associated V_{H} and V_{L} domains, (V_{H}·V_{L}), as well as single domain antibodies (V_{H} or V_{L}) are also intended to be encompassed by this invention. In addition, any other Ig Superfamily molecule having the requisite sequence and structural homology are also intended to be encompassed by this invention.

Alternatively, since the sequence would be known for the Ig superfamily CDR-like regions of interest, alignment procedures that relate tertiary to primary structure are useful to predict successful χ-proteins. Figures 13A (SEQ ID NOS: 10-16 and 18-30) and B (SEQ ID NOS: 1-9 and 31-40) depicts the amino acid residue alignment of Fv regions from mouse myeloma antibodies and other Ig superfamily molecules. As described in detail in Example 3, this type of sequence alignment allows approximate choice of splice points without having a three-dimensional crystallographic structure. The tertiary structures of framework regions are highly conserved, thus allowing reliable three-dimensional models to be predicated on sequence alignment methods.

However, to correctly splice "target" CDRs-like regions into the source Fv, some modifications to the source FR-like region sequence may be necessary. Moreover, it may be desirable to modify the native CDR-like sequence which is spliced into the source Fv, or the source Fv itself, to modify binding affinity or specificity. Such modifications are intended to be encompassed by the subject invention.

For example, one or more amino acid residues can be substituted by another amino acid of a similar polarity which acts as a functional equivalent, resulting in a silent alteration. Substitutes for an amino acid within the sequence may be selected from other members of the class to which the amino acid belongs, such as the nonpolar, polar, and positively or negatively charged amino acids.

The structure may be modified by deletions, additions, substitutions and insertions of one or more amino acids which do not substantially detract from the desired functional properties of the χ-protein. Naturally occurring allelic variations and modifications are included within the scope of this invention so long as the variation does not substantially reduce the ability of the χ-protein to bind its ligand.

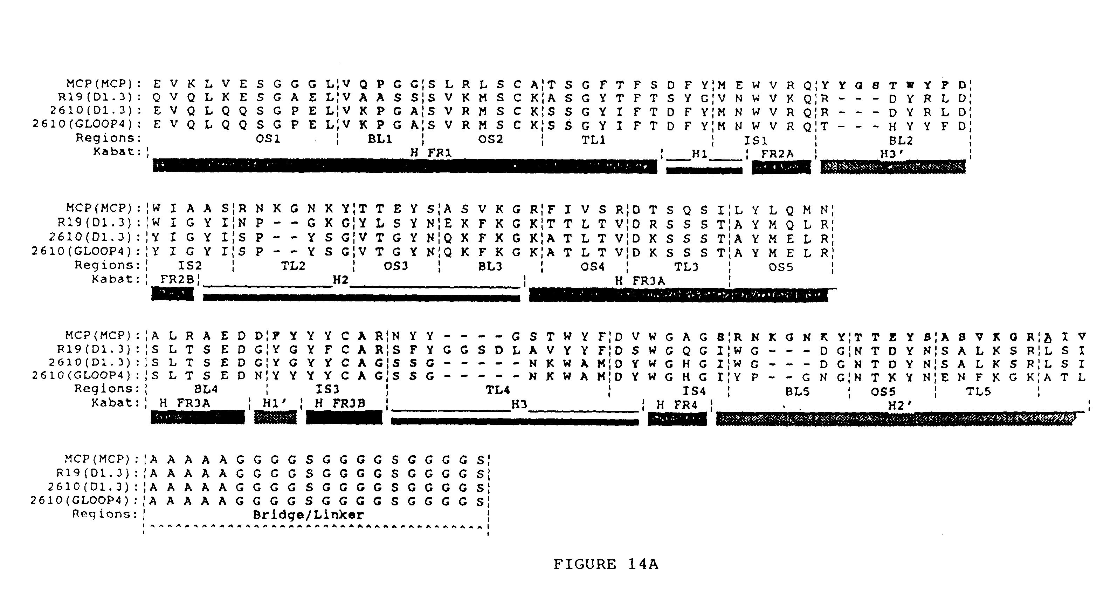

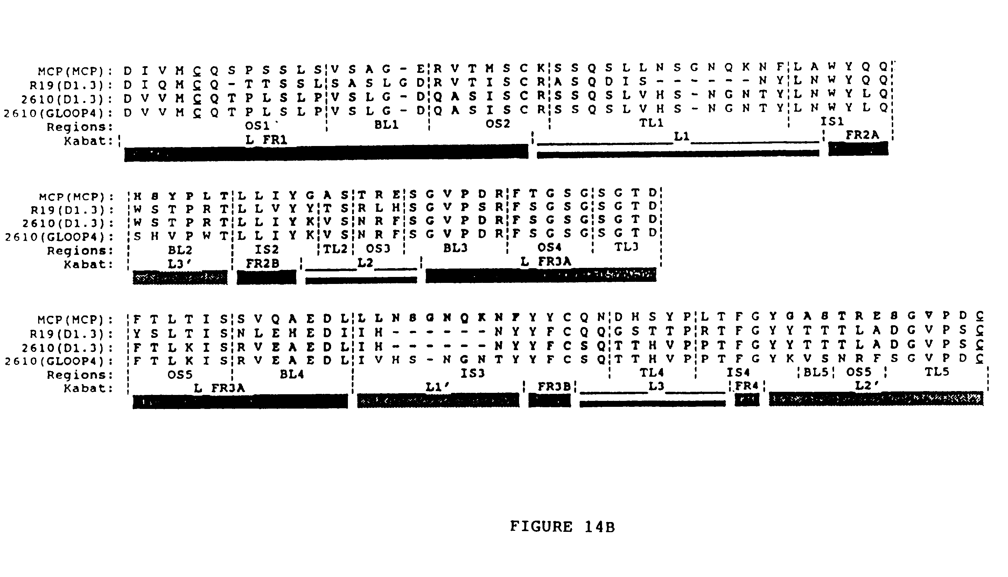

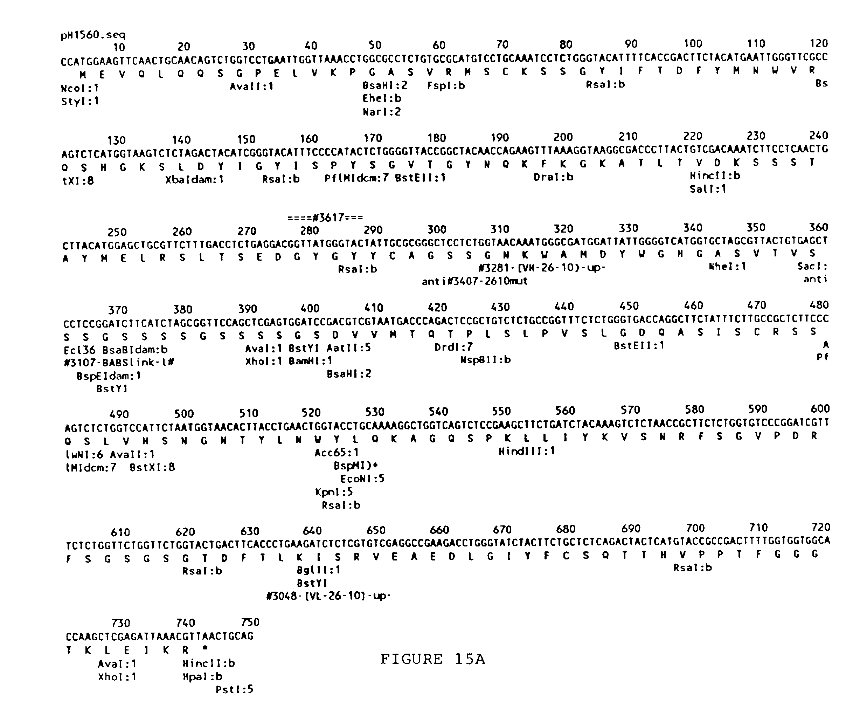

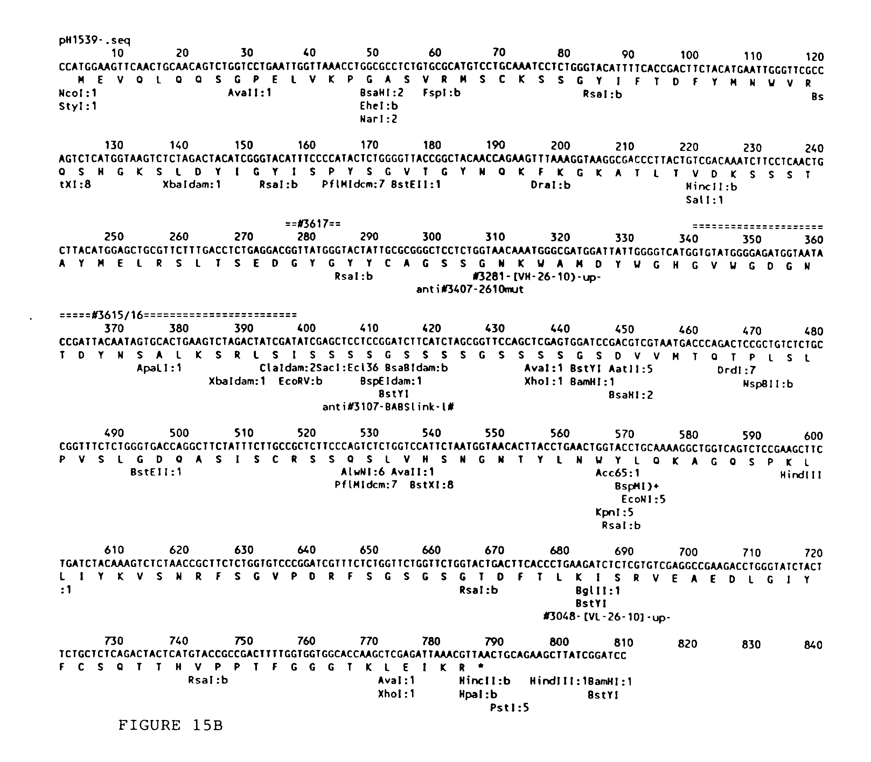

Based on the method described in Example 3, a χ-protein has been partially constructed incorporating two loops of an anti-lysozyme monoclonal antibody in the 26-10 sFv, as described in detail in Example 4. As shown in Figure 15A and 15B (SEQ ID NOS: 45-48), constructions have been made which incorporate the H1 and H2 loops of the D1.3 anti-lysozyme monoclonal antibody in the appropriate χ sites at the bottom of 26-10 sFv, where they are designated H1' and H2'. The design process has involved successive addition of χ-loops, so that there are constructions with H1' alone (X-1, Figure 15A), and H1' + H2' together (x-2, Figure 15B). In constructing χ-1 and χ-2, the only deviations from the corresponding partial constructs described in Example 3 (Figure 14, 2610(D1.3) sequence) are the following: (1) the isoleucine at the N-terminal end of H2' at V_{H} residue 112 is a valine in χ-2 due to limitations imposed by the restriction sites that were utilized, and (2) in both χ-1 and χ-2, the linker is (Ser₄Gly)₃Ser which reasonably confers better solubility properties on the proteins than that noted in Figure 14, Ala₅(Gly₄Ser)₃. The stepwise incorporation of the remaining D1.3 CDRs (H3', L1', L2', and L3') can be accomplished by the same procedure.

As each CDR loop was inserted in the χ-site, the cloned proteins were expressed in E. coli and refolded. The recovery of ouabain binding capacity by these χ-proteins demonstrates that the insertion of these χ-CDRs was compatible with proper refolding of the 26-10(D1.3) sFv polypeptide chain, thereby generating a 26-10 digoxin binding site in each case. (Ouabain is a digoxin analogue with Kₐ for association with 26-10 sFv of about 10⁷ M⁻¹, and ouabain-based affinity chromatography is the preferred isolation method for digoxin binding proteins.

Thus, experimental results support the rationale of inserting CDR loops in place of turns in β-sheet structure or near the C-terminal ends of V-regions. The maintenance of V region integrity after insertion of χ-loops is a fundamental premise for the construction of these dual binding site x-proteins. The ability to make a biologically-active χsFv protein (i.e., a χ-protein that retains its binding activity due to proper conformation) with 2 inserted loops (here comprising 24 CDR residues) is a remarkable result, in that the phage display manipulation of sFv binding sites may be used in entirely novel ways to genetically convert these added χ-site residues into virtually any desired antigen specificity. These results also reasonably support the anticipated ability to closely mimic a parent antibody combining site once more χ-CDRs have been incorporated.

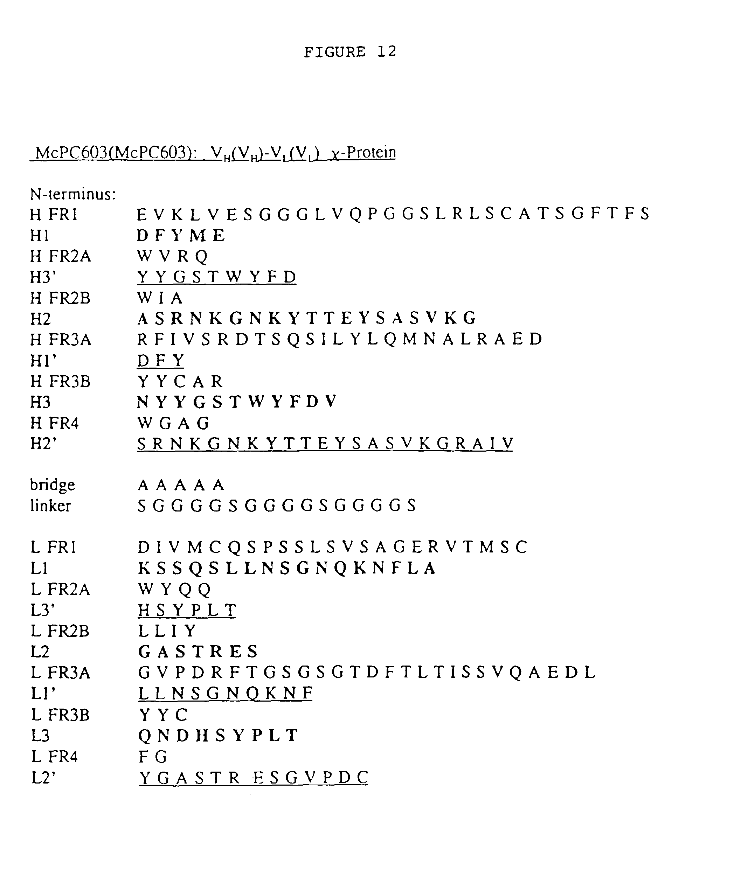

The present invention also relates to the amino acid sequences encoding the χ-protein. Once the optimal splice points for CDR-like region insertion have been determined and shown to adequately reproduce the native site, the linear amino acid sequence can then be deduced. The χ-protein, or its modified equivalent, can then be made by recombinant DNA methods or synthesized directly by standard solid or liquid phase chemistries for peptide synthesis, or other methods well known to one skilled in the art. For example, the amino acid sequence of the χ-protein McPC603(McPC603) shown in Figure 12 can be synthesized by the solid phase procedure of Merrifield by semisynthesis or enzymatic or chemical combinations of appropriately modified blocks of peptides.

The present invention also relates to nucleic acid sequences, DNA and RNA, that encode the χ-protein, and expression vectors capable of expressing the χ-proteins. Preferably, the χ-proteins of the subject invention will be produced by inserting DNA encoding the desired amino acid sequence of the χ-protein into an appropriate vector/host system where it is expressed, or the gene may be used in a cell-free rabbit reticulocyte ribosomal protein synthesis system.

A variety of host/vector systems can be used to express the polypeptides of this invention, such as the one described in Example 4. Primarily, the vector system must be compatible with the host cell used. Host/vector systems include, but are not limited to, the following: bacteria transformed with bacteriophage DNA, plasmid DNA or cosmid DNA, microorganisms such as yeast containing yeast vectors; mammalian cell systems infected with virus (e.g., vaccinia virus, adenovirus, etc.) or expressing plasmids; insect cell systems infected with virus, such as baculovirus or Xenopus oocytes injected with DNA encoding the χ-protein. Other methods well known to one skilled in the art may also be used.

Any of the standard recombinant methods for the insertion of DNA into an expression vector can be used. The recombinant vector can be introduced into the appropriate host cells (bacteria, virus, yeast, mammalian cells or the like) by transformation, transduction or transfection, depending upon the host/vector system and cultured to express the polypeptides of this invention.

Although strategies used to produce recombinant proteins have often relied upon bacterial expression of fusion proteins followed by in vitro refolding of the protein, direct expression and secretion also may be used to produce the χ-proteins of the subject invention. Expressed fusion proteins may require purification and possible removal of the leader sequence before refolding of the χ-protein to recover binding activity. However, this may be accomplished using routine laboratory procedures. Direct expression of the χ-protein can potentially produce the protein without the leader, but still in need of refolding, whereas secretion can ideally produce native protein for subsequent isolation. (Huston, J.S., et al. Methods Enzymol., 203:46-88 (1991)).

Alternatively, fusion proteins may be the end product of expression. For example, a cytochrome b₅ tail could be fused to the χ-protein (at the gene level), to make a membrane anchor that could hold the χ-protein in a membrane or Langmuir-Blodgett film. A tail or leader sequence could also include a specific recognition sequence for enzymatic or chemical modification. Such an engineered, post-translational modification could allow specific incorporation of moieties such as biotin or phosphoinositol.

Possible refolding protocols include dilution refolding, redox refolding and disulfide-restricted refolding. (Huston, J.S., et al. Methods Enzymol. 203:46-88 (1991) the teachings of which are hereby incorporated by reference). Dilution refolding relies on the observation that fully reduced and denatured antibody fragments can refold on removal of denaturant and reducing agent with recovery of specific binding activity. (Haber, E., Proc. Natl. Acad. Sci. U.S.A., 53:524 (1964).

Redox refolding utilizes a glutathione redox couple to catalyze disulfide interchange as the protein refolds into its native state. (Saxena, P. and Wetlaufer, D.B., Biochem., 9:5051 (1971); (Huston, J.S., et al. Methods Enzymol. 203:46-88 (1991)).

Disulfide-restricted refolding involves initial formation of intrachain disulfides in the fully denatured protein. This capitalizes on the favored reversibility of antibody refolding when disulfides are kept intact. (Buckley, C. E., et al ., Proc. Natl. Acad. Sci. U.S.A., 50:827 (1963); Huston, J.S., et al. Methods Enzymol. 203:46-88 (1991)). Disulfide crosslinks should restrict the initial refolding pathways available to the molecule. For chains with the correct disulfide pairing, the recovery of a native structure should be favored, while those chains with incorrect disulfide pairs must necessarily produce nonnative species on removal of denaturant.

Characterization of the multiple ligand binding sites requires that both their affinity and specificity be determined. Ideally, the measurement of binding affinity should use a thermodynamically rigorous approach such as equilibrium dialysis or ultrafiltration. In the absence of such methods, agreement between two distinct methods is desirable to reinforce the veracity of the data. Ligand binding to high affinity binding sites frequently involves very fast rates of binding and slow rates of dissociation. This characteristic makes measurement of their association constants amenable to routine immunoassay procedures, such as immunoprecipitation, radioimmunoassay and ELISA. (Huston, J.S., et al. Methods Enzymol. 203:46-88 (1991)).

After evaluating the binding activity of a particular model χ-protein, it may be desirable to further refine the initial χ-protein to enhance its biological activity. This refinement may be performed computationally by additional computerized modeling or it may be a "biological" refinement using recently developed techniques such as the use of genetically engineered bacteriophage to display and/or secrete the χ-protein and thereby select for an improved confirmation. (Marks, J.D., et al., J. Mol. Biol. 222:581-597 (1991)).

Additional optimizing techniques, as described above, may also be used to confer "special" properties on the χ-protein, such as "humanizing" the χ-protein using human FR-like regions as the scaffold protein to reduce the possibility of adverse immunologic reactions during therapy. (Daugherty, B. L., et al., Nucleic Acids Res. 19:2471-2476 (1991)). Special properties may also include increased solubility or stability or improved renaturability or secretion.

This invention further relates to the method of producing the χ-protein which includes the steps of determining the splice points for additional binding sites, as described in Examples 2 and 3, determining the amino acid sequence of the resulting construct, deducing the DNA sequence encoding that amino acid sequence, inserting the DNA sequence into an appropriate expression vector and expressing the protein in a suitable host system, refolding the protein to its biologically active conformation and analyzing its biological binding activity. For example, in the case of a χ-Protein with catalytic activity, this would include measurement of enzymatic properties.

The ability to target therapeutic agents in a host with antibodies has been a long-term goal of medical research. The term host, as used hereinafter, is intended to encompass mammalian hosts, including humans. The most elegant targetable proteins would consist of the minimum structures needed for selective delivery and effector function. (Tai, M., et al., Biochem. 29: 8024-8025 (1990)). Although the utility of binding proteins having multiple binding sites of different specificity has been widely recognized (U.S. Patent 4,676,980), these crosslinked antibodies or crosslinked antibody fragments suffer from the need for complex purification schemes and large molecular size.

Notwithstanding the construction of the smallersized sFv, the need still exists for a multifunctional binding protein with as reduced a size and as compact a shape as possible to permit effective therapeutic use. The subject invention also relates to use of these χ-proteins in therapeutic and diagnostic procedures. These uses include, but are not limited to, in vivo and in vitro imaging agents, delivery agents for drugs, radioisotopes, and cytotoxic substances. The χ-protein could also include a binding site for an effector molecule, such as an enzyme, growth factor, celldifferentiation factor, lymphokine, cytokine, hormone, anti-metabolitic, or an ion-sequestering sequence such as calmodulin. The χ-protein could further include a binding site reactive with antibody-dependent cytolytic cells or cytotoxic T cells. Also included are χ-proteins with catalytic or biosensor activity, as well as binding sites that facilitate affinity purification procedures.

In some instances, the primary binding site could be a high affinity site that targets the protein to specific cell surface locations, and the secondary site designed to decrease normal receptor activity. A χ-protein could be designed with one binding site comprising a receptor for a cell surface antigen and a second binding site comprised of a modified receptor with diminished affinity for its ligand. Thus the χ-protein would to bind a cell via the normal binding site, leaving the binding site with diminished binding activity exposed, thereby resulting in decreased receptor activity. For example, a χ-protein could be designed with neural cell adhesion molecule (NCAM) variable regions that would modulate cell interactions such as contact inhibition of malignant cells.

A χ-protein may also be designed to modify, enhance or inhibit cell-cell interactions, or communication. For example, one binding site could be designed to target a cancer cell and a second binding site could be designed to target an effector cell, such as a macrophage, thus binding the macrophage in a manner which results in destruction of the targeted cell.

Moreover, a χ-protein could mediate phenomena such as antibody-dependent cellular toxicity. (Huston, J.S., et al., Proc. Natl. Acad. Sci. USA, 85:5879-5883 (1988)). For example, a χ-protein could be designed with one binding site comprised of a receptor for a cell surface marker protein and a second site derived from a receptor for killer T cells. The χ-protein would bind to the target cell and the secondary site would bind the cytotoxic T cell, resulting in destruction of the target cell. The χ-proteins of the present invention would exhibit significantly greater tissue accessibility or tumor penetration and faster pharmacokinetics due to their compact size.

Recently, it has been shown that an Fv undergoes a conformational change upon antigen binding. (Bhat, T. N., Nature, 347:483 (1990)). It is reasonable to predict, that the χ-proteins of the present invention would undergo similar conformational changes, due to the significant sequence and structural homology with the native Fv. Although this conformational change is modest, it involves the translational movement of the V_{H} and V_{L} domains relative to each other, such that the orientation of the bottom loops can shift. This makes the χ-protein conformation sensitive to binding at either the first or second binding site, and requires that linkage exists between their binding equilibria. It is reasonable to predict that this conformational change would enable the χ-protein to act as a "molecular switch".

For example, the first site of the χ-protein may be a catalytic antibody combining site (e.g., a site that catalyzes the conversion of a "pro-drug" to a cytotoxic drug), while a second site is specific for binding to a marker on a cell surface (e.g., a tumor cell). If binding to the cell surface epitope induces a conformational change in the χ-protein such that the loops of the catalytic site are brought into optimal geometry for efficient catalysis, then the pro-drug would be converted to cytotoxic drug directly at the site of the tumor, so that high cytotoxic levels could be maintained at the site while serum levels remained low. The unbound catalyst would have low efficiency and, because of its low molecular weight, would clear rapidly from circulation.

The χ-proteins are well suited to applications in immunotargeting because of their binding capabilities and compact size. The absence of constant domains will reduce nonspecific background binding, thus enhancing visualation of target tissue by in vivo or in vitro imaging procedures. The use of χ-proteins in in vitro diagnostics would also reduce nonspecific binding thereby increasing the accuracy of these assays.

The χ-proteins of the present invention are also useful to deliver drugs, cytotoxic substances or effector molecules to immunotargeted tissues. One of the binding sites of the subject protein may exhibit catalytic activity that is triggered by binding of a specific ligand to the other binding site.

The invention will be further illustrated by the following Examples, which are not intended to be limited in anyway.

### Example 1

### Constructing a Model of Bivalent McPC603 χsfv

The following illustration, which utilizes the McPC 603 Fv region (SEQ ID NOS: 1 and 10), for which the three-dimensional crystallographic structure has been determined, has been chosen in order to assess how well a particular set of splice points will generate a χ-site (additional binding site) that recreates the parent binding site. This example also reveals the general method of building a χ-protein model, by the specific example of mapping the McPC603 V_{H} CDRs to V_{H} bottom loop positions, and the V_{L} CDRs to V_{L} bottom loop positions. Molecular modeling was performed on a Silicon Graphics 4D/70GT Superworkstation using the Biosym programs INSIGHT (to visualize models), HOMOLOGY (to assemble spliced CDR/FR sequences), and DISCOVER (to minimize the energy of the model) (Biosym, Inc., San Diego, CA). The HOMOLOGY program was used to assemble the structure in five steps, and the DISCOVER program was used to minimize the energy of the resulting model about the splice points. Although, HOMOLOGY was used in this example, this program is not necessary to build the model. Any one of several alternative molecular mechanics programs, such as AMBER and CHARMM, could be used instead of the DISCOVER program.

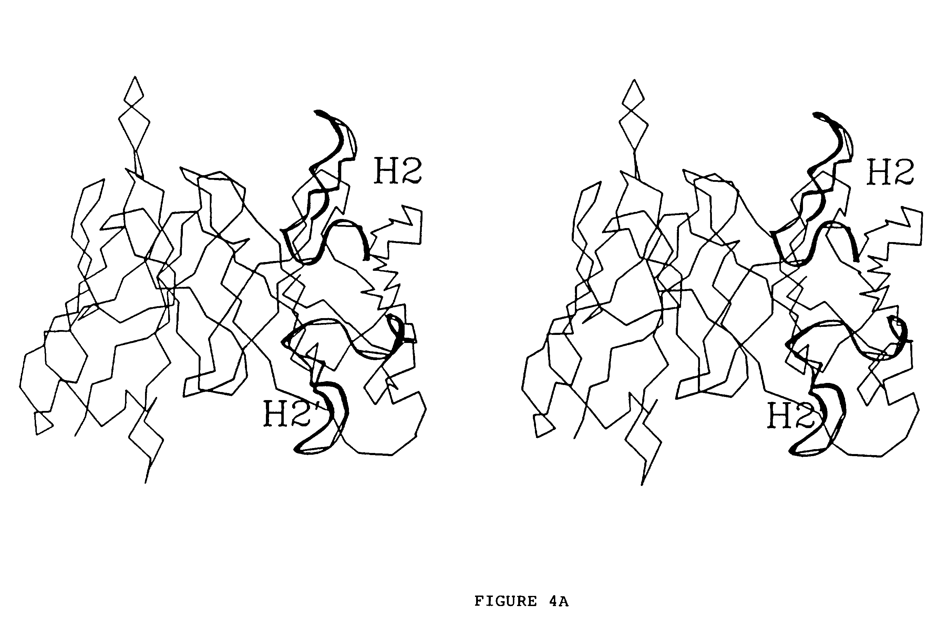

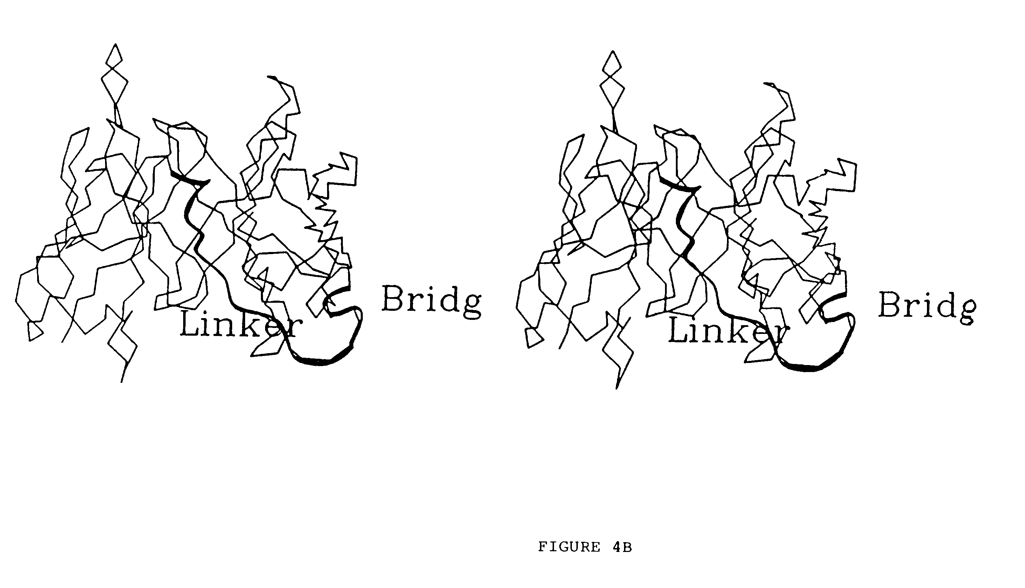

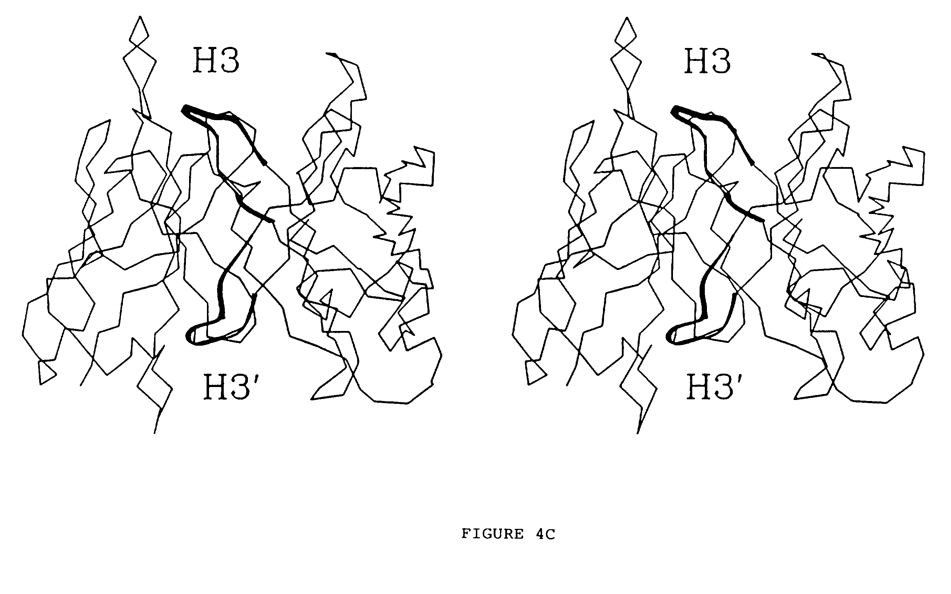

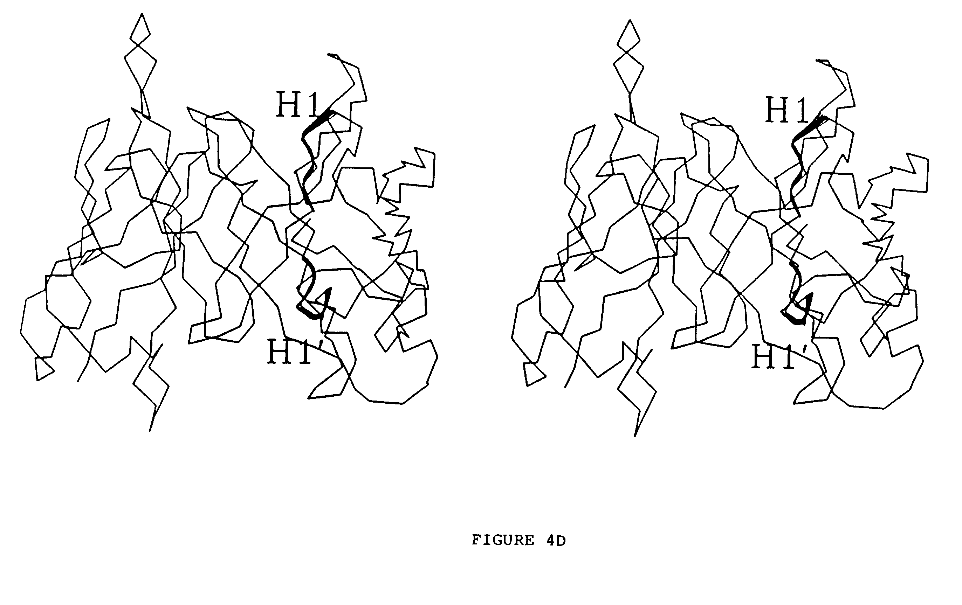

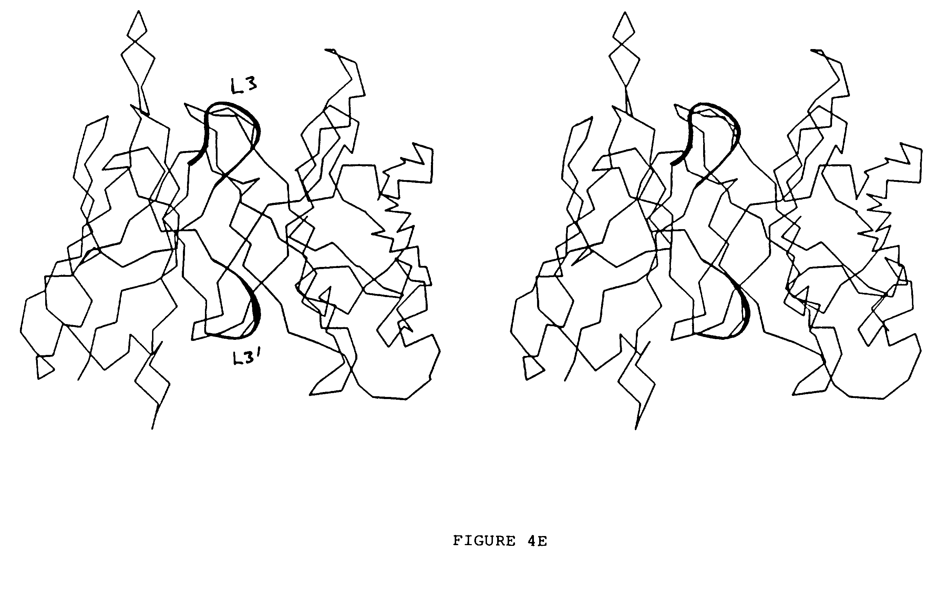

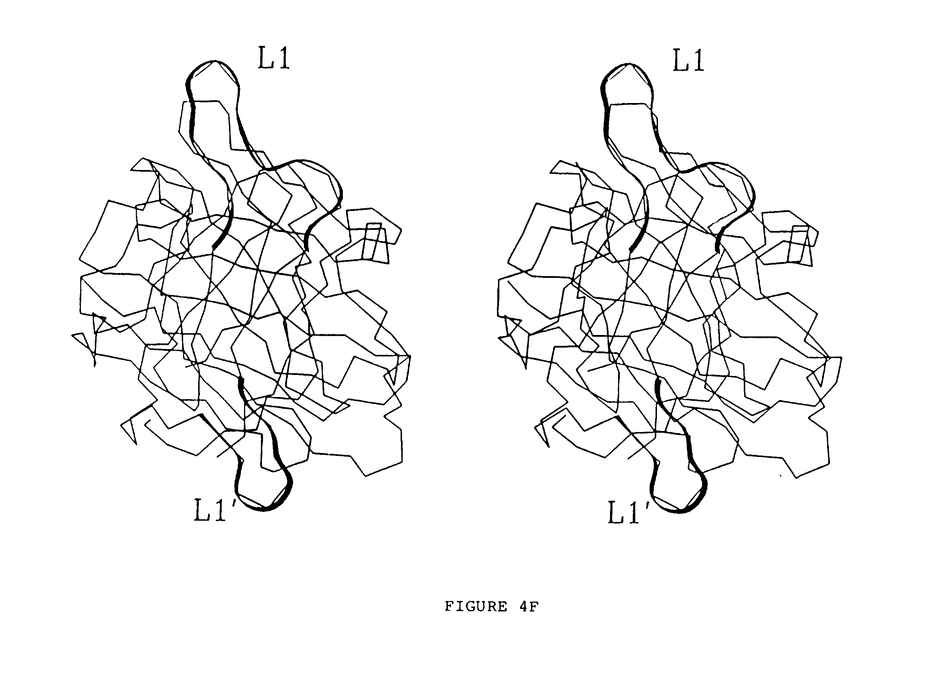

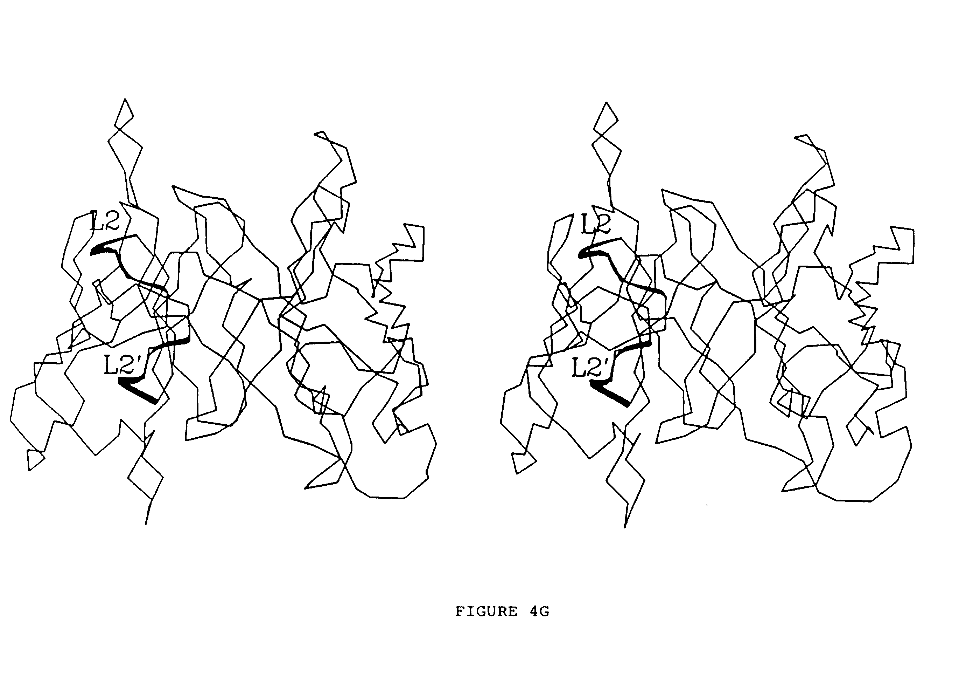

Two copies of the McPC603 sFv structure were superimposed (Figure 3A and 3B): model A had the binding site up and the model B had the bottom loops up; the CDR loops of B superimposed on the lower loops of A, as described above. The primed loops (e.g. H2') are those spliced into the χ-site.
1. Building the H2' loop and the bridge. (Figures 4A and 4B). The A coordinates for the V_{H} domain were used up to where H2' from structure B splices into the C-terminus of structure A and the A coordinates of the linker and the following V_{L} domain were likewise used. The coordinates of the H2' segment were taken from B. Once coordinates had been assigned to the residues flanking the bridge peptide region, the HOMOLOGY loop search algorithm was used to find a 5 residue peptide whose flanking 3 alpha carbons overlapped well with the 3 amino acids on either side of the gap, and whose conformation best fit the surface of the V_{H} domain. The HOMOLOGY program automatically connects the peptide bonds at the splice points to create the new single chain structure called MDL1. It will generally be necessary to rotate side groups in the interface between V_{H} and H2' out of the way in order to reduce steric conflicts. One residue in H2' had to be changed because simple rotation could not eliminate its steric conflicts: Phe 137 (position H70)→ Ala. Using the DISCOVER program, the atoms about the splice points were moved so as to minimize contributions to the energy of the structure due to improper bond lengths and geometries. At this point the composition of bridge peptide was polyalanine; additional computational analysis of the model may be used to further assess the optimum length, path and composition of the bridge. This new structure (with H2' and the bridge) is called MDL1.
2. Building H3' and H1' Loops. (Figures 4C and 4D) Structure MDL1 supplied the coordinates for the residues preceding and following the segment where H3' was to be built into the model. The coordinates for the H3' segment were taken from structure B. Since H1' was a short segment of 3 residues that would replace exactly 3 residues of MDL1, H1' was built by side chain substitution. This structure is MDL2.
3. Building L3'. (Figure 4E) Structure MDL2 supplied the coordinates for the residues preceding and following the segment where L3' was to be built into the model. The segment for L3' was built into the next stage of the model in a manner analogous to the way that MDL2 was built. The resulting structure is MDL3.
4. Building L1'. (Figure 4F) Structure MDL3 supplied the coordinates for the residues preceding and following the segment where L1' was to be built into the model. The segment for L1' was built into the next stage of the model in a manner analogous to the way that MDL2 was built. This structure is MDL4.
5. Building L2'. (Figure 4G) Structure MDL4 supplied the coordinates for residues preceding the segment where L2' was to be built into the C-terminus of the model. The segment for L2' was built into the next stage of the model in a manner similar the way that MDL1 was built except that no connection (bridge or linker) was made to the C-terminus of the L2' segment. In order to stabilize the C-terminus of the L2' loop, a disulfide bridge was constructed between the C-terminus of L2' and the segment that derived from the V_{L} N-terminal strand by substituting the Arg in L2' (at the position corresponding to L68 in V_{L}) and the Thr at the position corresponding to L5 in V_{L} with Cys.
6. Refinement. At each stage, atoms making bad contacts were rotated out of the way. This often required breaking the peptide bond between neighboring residues, rotating a backbone bond and then reforming the peptide bond. The functionality for these modeling operations is built into the INSIGHT program and other similar molecular modeling programs. The resulting modified peptide bond was added to the list of splice points. Strain in the splice point peptide bonds was minimized by subjecting the residues on either side of the spliced peptide bond to 100 cycles of steepest descent minimization using the DISCOVER molecular mechanics program. The final structure was minimized for 1000 steps of steepest descent minimization without coulumbic interactions, followed by 200 steps steepest descent, and 1000 steps of conjugate gradient minimization with columbic charge interactions.

Figures 4A-4G show the final model of the McPC603 (McPC603) V_{H}(V_{H})- V_{L}(V_{L}) χ-protein with corresponding pairs of loops highlighted with ribbons. The symmetry between the top and the bottom of the molecule can be seen. Each of the 5 pairs of spliced loops are highlighted in separate frames.

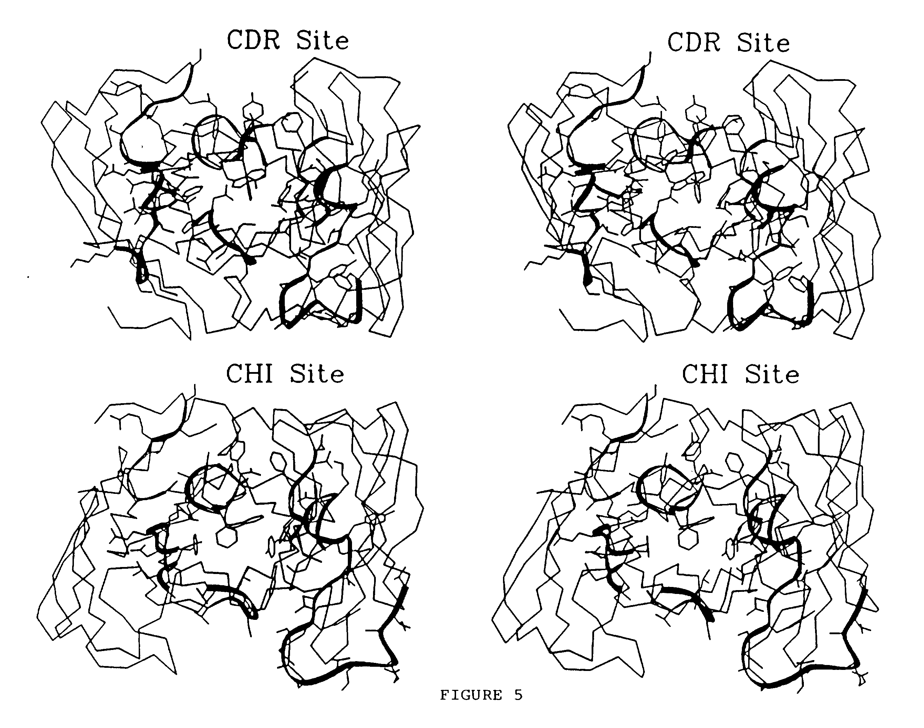

A detailed comparison of the conformation of the new sites is presented in Figure 5. The upper view is of a first combining site on the top of the molecule while the lower view is the χ-site built onto the bottom of the molecule. In the figure, corresponding regions of the loops are highlighted and side chain heavy atoms are shown. It can be seen that during the minimization process, there was some divergence in the conformation of the backbone and of the side chains between these two sites, but there still remains an impressive degree of homology between them. This is a measure of the congruency of the lower loop stems and the corresponding upper loop stems. The observed homology also suggests that the steric environment surrounding the spliced loops must be similar to that of the parent loops, even though it is clear that the loops of the first site are more exposed than are those of the second χ-site.

### Example 2

### Defining top and bottom loop symmetry and choosing splice points.

Because of the high level of symmetry that exists between the V_{H} and V_{L} subunits, CDR loops can be spliced in a "homologous" fashion (V_{H} top loops onto V_{H} bottom loops, V_{H}(V_{H}), and V_{L} top loops onto V_{L} bottom loops, V_{L}(V_{L})) or in a "heterologous" fashion (V_{L} top loops onto the bottom of V_{H}, V_{H}(V_{L}), and V_{H} top loops onto the bottom of V_{L}, V_{L}(V_{H})). In addition, there are two ways to connect the V_{H} and V_{L} regions: 1) linker peptide bridging from the C-terminus of V_{H} to the N-terminus of V_{L} (V_{H} - linker - V_{L}), or 2) linker peptide running from the C-terminus of V_{L} to the N-terminus of V_{H} (V_{L} - linker - V_{H}). There are, therefore, four possible types of χ-protein derived from immunoglobulin CDRs.

If one is splicing the CDRs of one V_{H} into the bottom loops of another V_{H} (as in Example 1) then H2 would be spliced into the V_{H} C-terminal strand. The C-terminal end of the new H2' segment would end up on the outside of V_{H} region, near its N-terminal strand (Figure 3). Thus, if one is splicing heavy chain loops into the bottom of the heavy chain in a sFv connected in the V_{H} - V_{L} order, the C-terminal end of H2' can be connected to the peptide that links the heavy and light chains. The L2 loop structure can likewise be spliced onto the C-terminus of V_{L}.

### Modeling a V_{H}(V_{H}) - (V_{L}-V_{L}) χ-Protein based on McPC603:

The following discussion presents a detailed analysis of how to construct a second McPC603 antigen binding site on the bottom of McPC603 to form a homo-domain V_{H}-linker-V_{L} χ-protein. Although this exemplification is based on McPC603, the structural homology that exists between the framework regions of known Fv structures clearly suggests that CDR splicing to the bottom loops of Fv regions will work with any antibody Fv. The existing structural homology between known structures permits one to structurally align the sequences of these known structures and to establish a numbering index wherein the position numbers in the strand regions identify unique structural locations. The position scale used to refer to splice points is defined in Figure 2B.

### Determining loop splice points

When the CDR loops are spliced into the lower loops, it is important to preserve those framework residues bordering both the top loops and the bottom loops which are critical for maintaining the proper folding of the β-barrel structure. In the following figures, the spatial alignment of bottom loops with the superimposed CDR loops are shown along with the segment of aligned sequence that derives from the chosen splice points. Three considerations govern the choice of the splice points:
1. Preservation of critical framework residues.
2. Maximal incorporation of CDR sequences.
3. Fusion at closet possible splice points. In order for the spliced loops to maintain their native structure in the new site, it is important that the chains be distorted as little as possible at the splice sites. Therefore one tries to chose splice points as close as possible to the positions where the aligned chains are closest together, e.g., where corresponding alpha carbons are separated by no more than a few angstroms.

### Determining the Splice Points for Splicing H3 (H-TL4) into H-BL2, and L3 (L-TL4) into L-BL2:

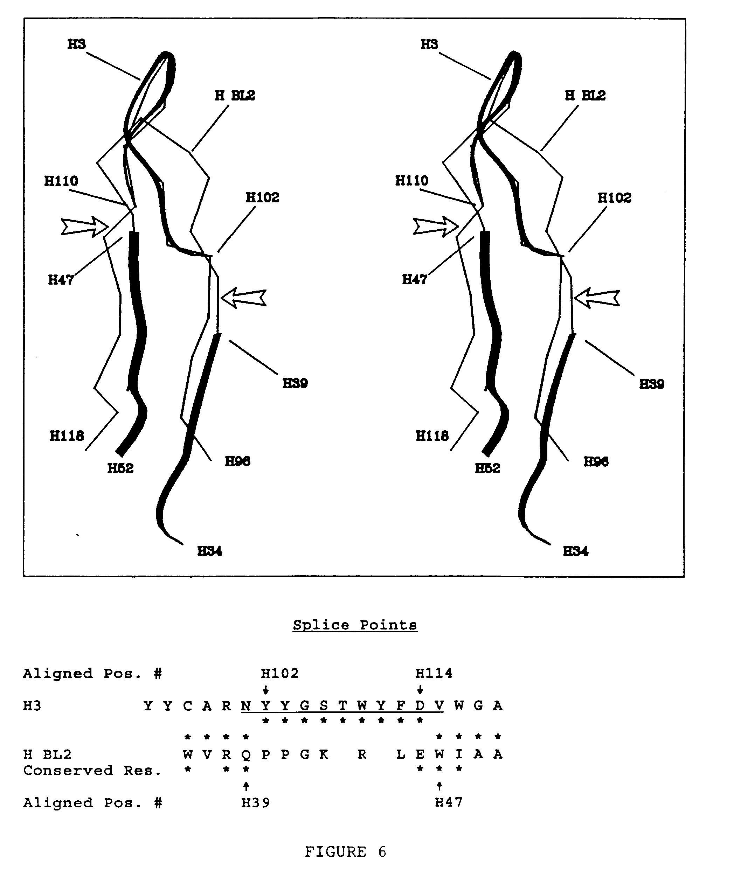

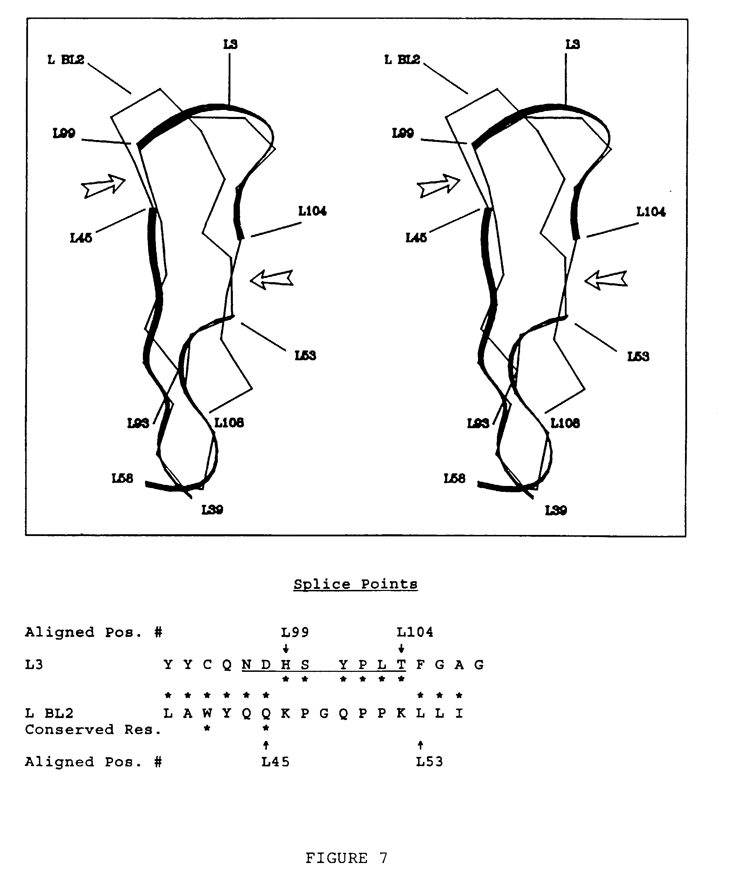

Figures 6 and 7 show how the CDR 3 regions (H3 and L3) would be spliced into H-BL2 and L-BL2, respectively. Critical framework residues H-BL2 and L-BL2 include the Gln at position H39 in H-BL2 and the Gln at position L45 in L-BL2.

Referring to Figure 6, residues position H102 to H114 from H3 (positions H101 to H115) can be spliced into H-BL2. The H3 residue at position H101 is not included because it would replace the critical framework residue at position 39. The H3 residue at position H115 is not included because splicing from position H114 in the H3 loop to position H47 in the framework leads to less distortion of both the framework and the loop conformations.

The L3 loop (positions L97 to L104) can be spliced into L-BL2 in a similar manner. Referring to Figure 7, the new L3' loop includes residues from positions L99 to L104. The L3 residues at position L97 to L98 are not included because L98 would replace the critical framework residue at position L45. The two chains are sufficiently close together at L3 position L104 that the entire C-terminal end of L3 can be spliced into the new L3' loop. Consequently, only one residue from the N-terminal end of L3 cannot be spliced. In the cases of both the H3' and the L3' splices, the backbones of the respectively aligned chains lie close enough together at the indicated splice points that there is little distortion of the backbone conformation in the χ-protein.

### Determining the Splice Points for Splicing H1 (H-TL1) into H-BL4, and L1 (L-TL1) into L-BL4:

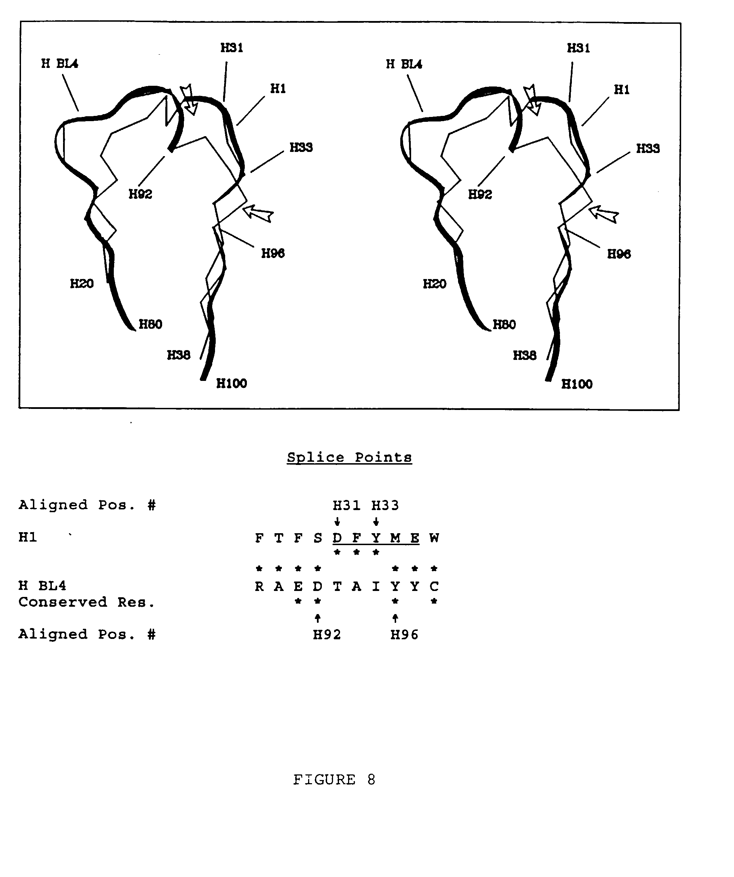

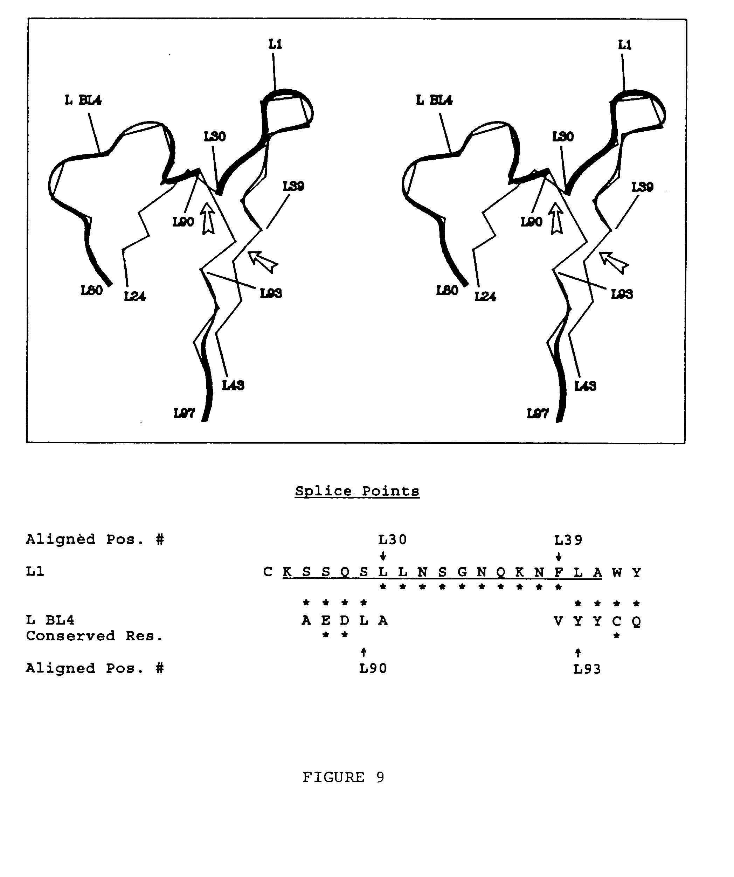

Figures 8 and 9 show how the CDR1 regions would be spliced into the fourth bottom loops. Because sections of both H-BL4 and L-BL4 that contain non-critical residues do not align well with the CDR1 portion of either CDR loop, only portions of the CDR1 loops can be spliced.

The H1 segment (positions H31 to H35) is 5 residues long in McPC603, and of these, the 3 residues (positions H31 to H33) that can be spliced are on the outer portion of H-TL1, which faces the binding site. Position H92 in H-BL4 is a critical residue that is involved in a conserved intrachain salt bridge, while positions H96 to H98 are conserved inner strand residues. Consequently positions H31 to H33 are spliced between positions H92 and H96 in H-BL4 to create H1'.

In McPC603, L1 (positions L25 to L41) is fairly long (17 residues), but only the internal 10 residues (position L30 to L39) can be spliced (Figure 9). Position L30 is the first place where the L1 loop and L-BL4 align. Framework positions L93 to L95 are conserved, inner strand residues that are structurally analogous to H96 to H98. In order to preserve framework position L93 the L1 loop is spliced at position L39. Consequently, 10 residues from the McPC603 L1 region can be spliced into L-BL4. In other antibodies having shorter L1 regions, only about 3 or 4 residues can be spliced.

### Determining the Splice Points for Splicing H2 (H-TL2) into the C-terminal end of VH:

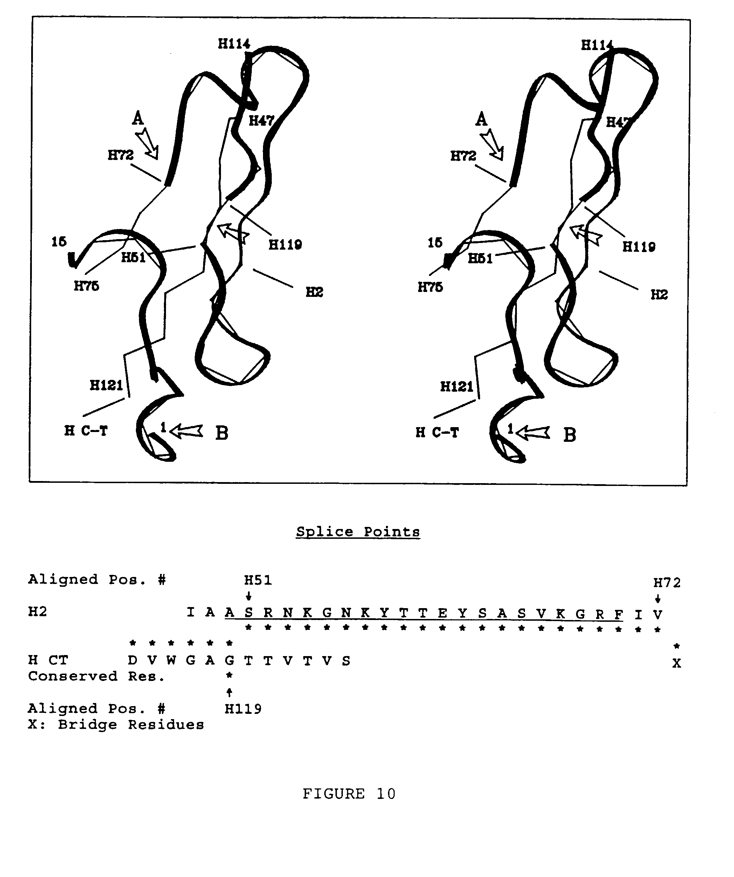

Figure 10 shows how the H2 region of the inverted copy of McPC603 aligns with the N-terminal and the C-terminal ends of V_{H}. Residues positions H48 to H50 that flank the N-terminal end of H2 loop region (positions H50 to H70) align closely with V_{H} C-terminal residue positions H117 to H119, respectively. When splicing in the H2' loop, one wants to preserve as much of the V_{H} C-terminus as possible. Based on the superposition of the C-terminal and H2' backbones, splicing can be done between positions H115 and H119 in the C-terminal strand and between positions H47 and H51 in N-terminal flanking sequence of H2. Because several residues in the C-terminal sequence of V_{H} (and V_{L} as well) are probably important to stabilizing the packing of BL1 and BL4, the splice should be made so as to preserve as much of the C-terminus as possible. As shown in Figure 10, therefore, the splice is made between position H119 of the V_{H} C-terminal strand and position H51 of the H2' loop. The C-terminal end of H2' (arrow A, Figure 10) is located on the surface of V_{H}, near the N-terminal strand of V_{H}, when H2' is properly positioned relative to H3', L3', H1' and L1'. To connect this end of the N-terminal end of the interchain linker used to create an single chain Fv (arrow B, Figure 10), a 5 residue bridge peptide must be added (between points B and A in Figure 10). In this arrangement, the linker-bridge structure folds across the bottom of the V_{H} chain. The preferred bridge peptide would contain Ser for solubility and non-polar residues to help anchor it to the surface of V_{H} so as to stabilize the conformation of the H2' fold. In the model and in the sequence shown in Figure 12, a polyalanine bridge has been used.

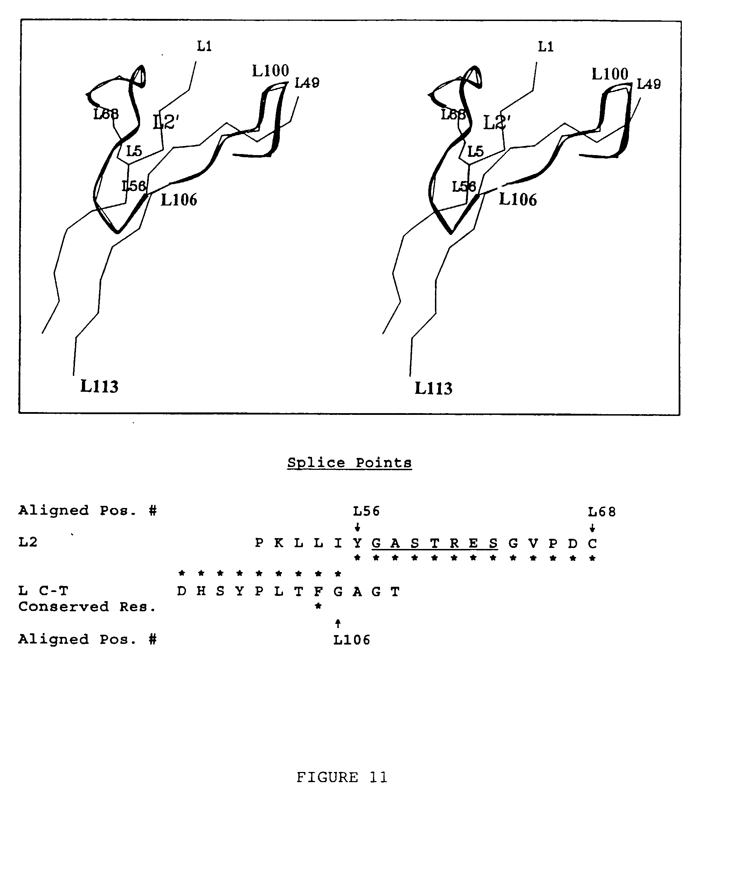

Figure 11 shows how the region of the inverted copy of McPC603 aligns with the N-terminal and C-terminal ends of V_{L}. Residue positions L49 to L57 that flank the L2 loop region (positions L57 to L63) align closely with V_{L} C-terminal residue positions L102 to L109. The same considerations are involved in making the L2' splice as with the H2' splice. The splice is made from C-terminal position L108 to L2 position L56. The L2 loop structure folds around until it runs parallel to the V_{L} N-terminal strand. In order to stabilize the L2' loop structure, we include a disulfide bond from the end of the L2' loop to a residue in the N-terminal strand of V_{L}. To accomplish this the Arg at position L68 in the C-terminal end of the L2 loop and the Thr at position L5 in the N-terminal strand of V_{L} were changed to Cys.

Figure 12 shows the sequence (SEQ ID NO: 17) of the resulting construct with the sequences of the primary CDR sites (bold) and secondary CDR sites (Underlined) identified.

### Example 3

### Determining χ-Site Splice Points From Primary Sequence Alignment

The members of the Ig Superfamily share a high degree of structural homology. By using the homology that exists between members of known structure, it is possible to engineer χ-proteins from Ig Superfamily molecules whose sequence is known but whose tertiary structure is not yet determined by crystallographic or NMR methods.

Figure 13A (SEQ ID NOS: 10-16 and 18-30) and 13B (SEQ ID NOS: 1-9 and 31-40) shows the set of sequences of Fv domains for which structures are known and are available from the Brookhaven Protein Databank (PDB at Brookhaven National Laboratory).

Table 1 lists the sequence and structure identification names, along with the Kabat et al. classification and references for the sequences that appear in Figures 2B (SEQ ID NOS: 1-16) and 13A (SEQ ID NOS: 10-16 and 18-30) and 13B (SEQ ID NOS: 1-9 and 31-40). The alignment of these sequences is based on the structural homology that exists between them, especially in the β-strand regions (OS and IS). Because of this high degree of structural correlation, one can assign positions in a "consensus 3-D model" to residues in a set of aligned β-barrel sequences. Furthermore, at certain positions, there exists a strong preference for certain residues or for residues having certain physical characteristics (Table 2). Thus, while the 3-D structures of most Fv sequences remain undetermined experimentally, the residue preferences at these positions makes it possible to reliably align these sequences with those V or Ig Superfamily sequences for which the 3-D structures have been experimentally determined. Ig V regions can thereby be aligned with the sequences of more distant members of the immunoglobulin superfamily, such as the T-cell receptor which was successfully modeled according to the McPC603 Fv structure (Novotny, J. et al. Proc. Natl. Acad. Sci USA 88:8646-8650 (1991).

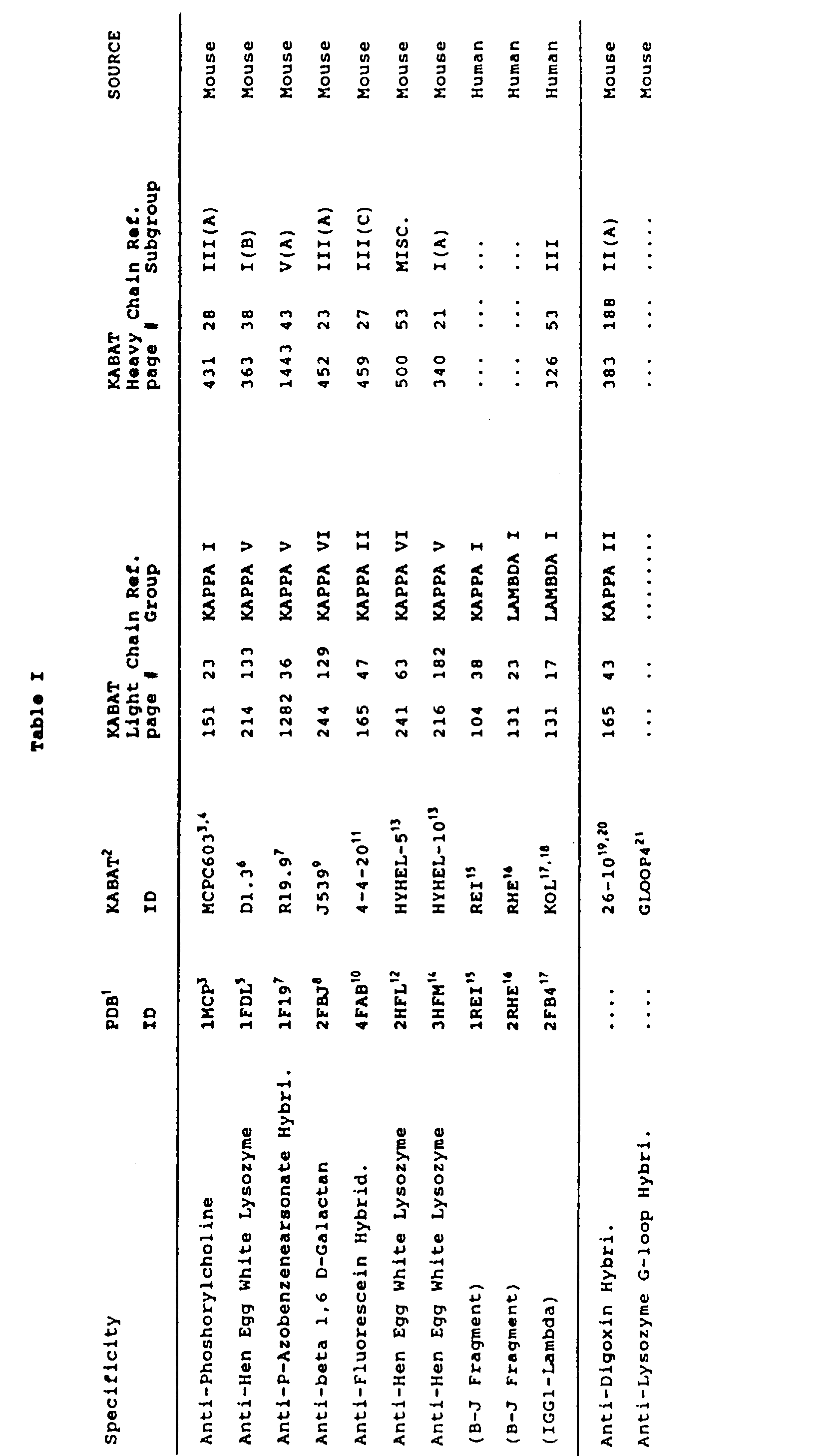

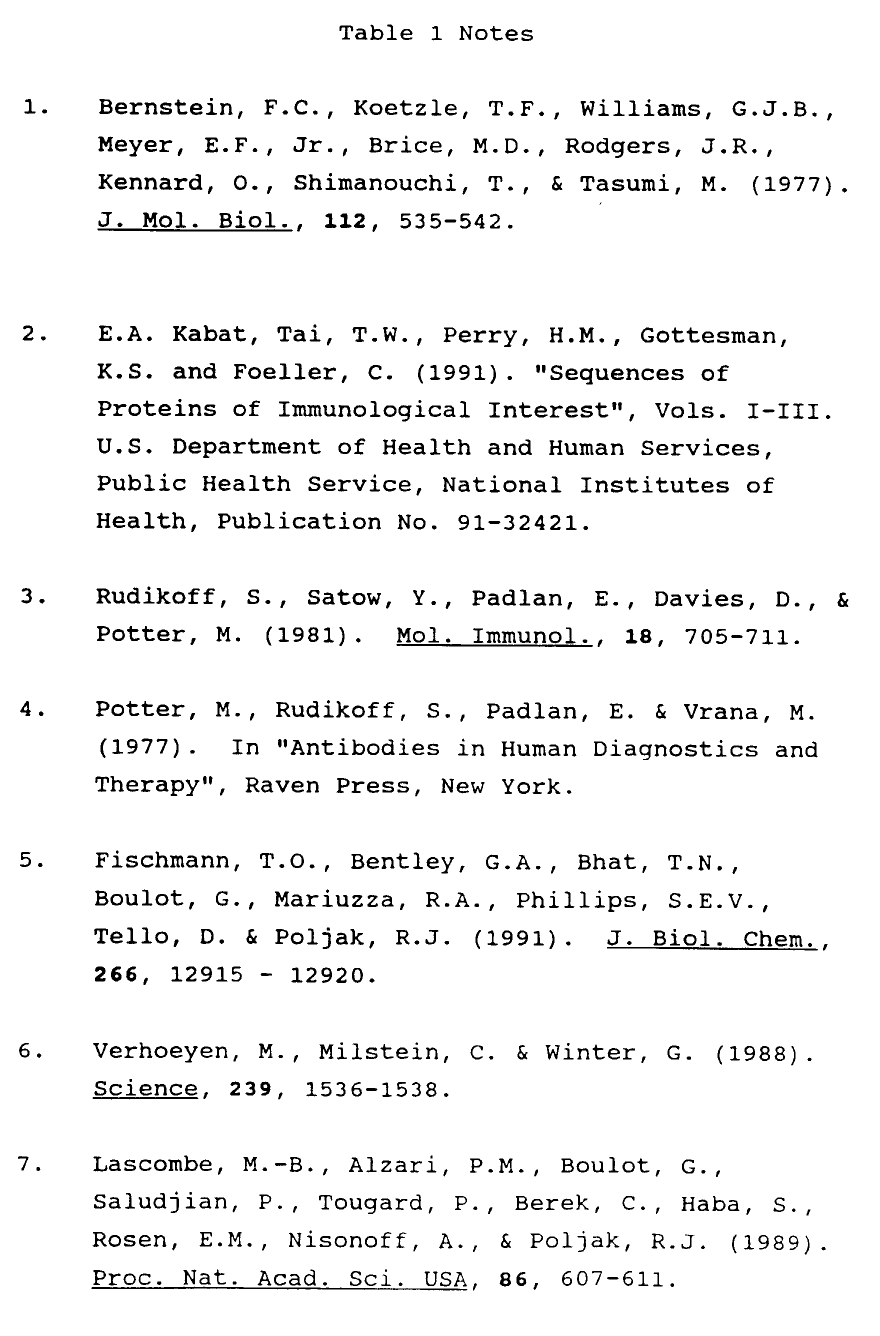

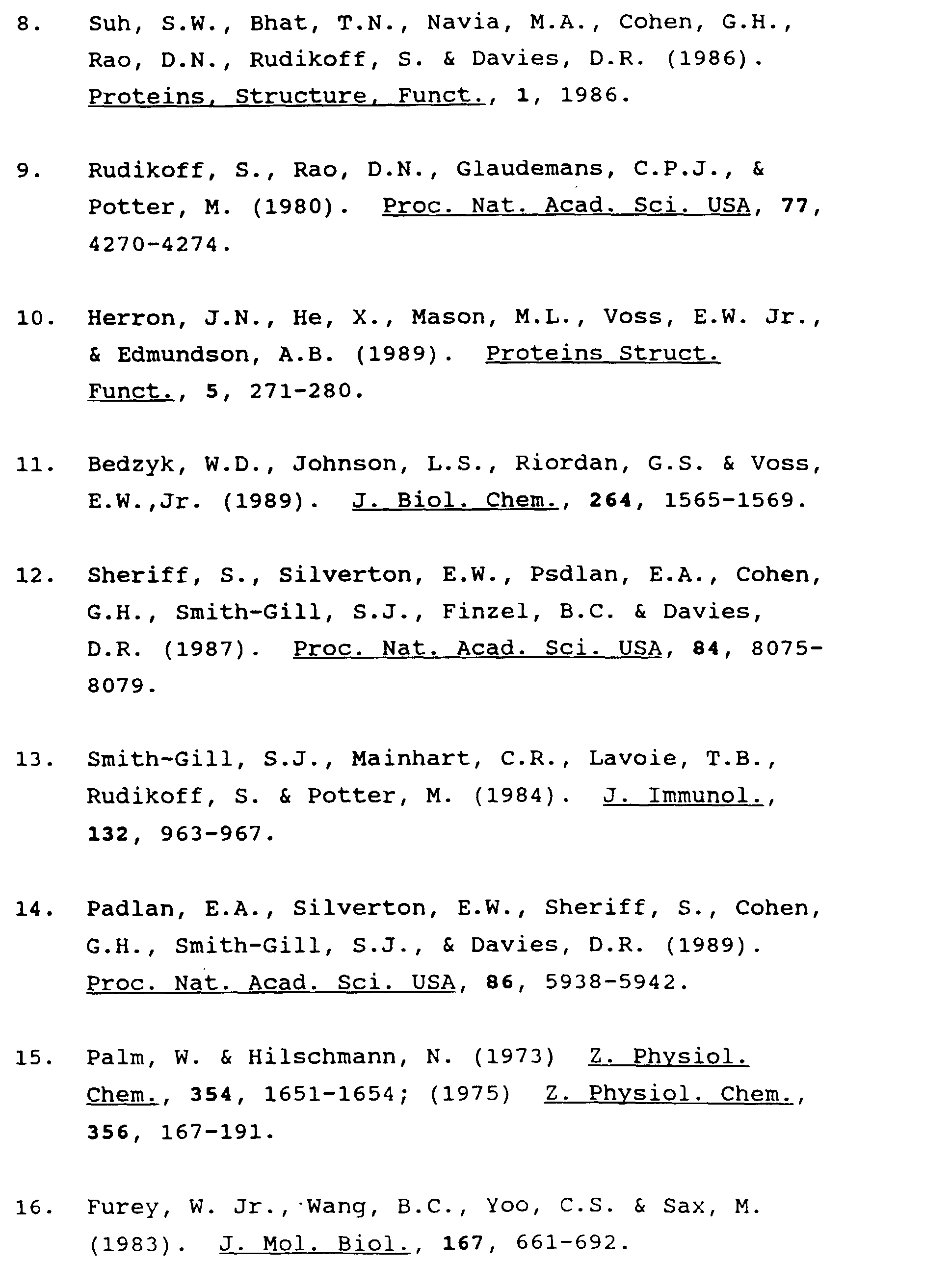

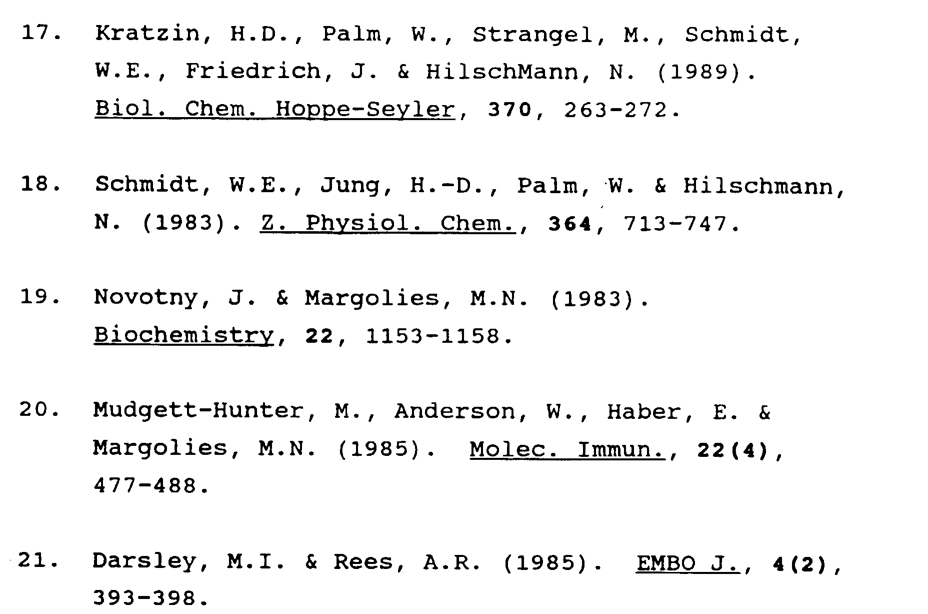

Once a sequence has been positioned in relation to the set of structurally aligned V region sequences, one can use the splice point analysis developed in Example 2 to construct a model and delineate a sequence for a χ-protein by analogy. The procedure for determining proper slice points is as follows:
1. Sequences of undetermined 3-D structure are lined up with the set of structurally aligned sequences. The sequence alignment process makes use of the conserved and semi-conserved residue positions listed in Table 1 to fix the alignment in certain regions. Between these *anchor points* it may be necessary to introduce gaps. Sequences in the β-strand regions (OS and IS) generally line up without the need for introducing gaps except in the N-terminal section of the light chain variable region (L OS1 - L BL1). Relative to all other light chain V region classes, kappa light chain class V sequences have a deletion at position L7 and an insertion at position L17. Lambda light chain sequences also have a deletion at position L7, but no insertion at position L17. Kappa light chain class VII sequences have a deletion at position L23. Other than these gaps in the N-terminal section of the light chains, the alignment of sequences is may make use of conserved positions to establish anchor points. Further help in the alignment of intervening sequences is derived from conservation of sidechain properties (i.e., polar, non-polar, charged, acid, basic, etc) at certain positions. The other major gaps will exist in the CDR loop regions (H1, L1, H2, L2, H3 and L3). Because CDR loops are variable in sequence and length, the exact location of gaps in these regions is less critical as the sequences in these regions show little homology. Structural variation tends to be the largest at the center of CDR loops of similar sequence, so gaps are preferably positioned in the center of CDR top loops.
2. Once the sequences of one or more V regions of undetermined structure have been lined up with the structurally aligned reference set, structure positions are assignable to the residues that locate in the regions of the inner and outer β-strands. Because the χ-site splice points occur near the ends of the β-strand regions, they can be assigned by analogy with the splice positions determined for McPC 603 in Example 2. Given two sequences aligned with the reference set, the primary sequence is that into whose bottom loops the χ-site will be built, and is referred to below as the *target* sequence; while the secondary sequence contains the CDR loops which will be built into the χ-site, and which will be referred to below as the *source* sequence. χ-site loops are created by splicing top loop segments from the source sequence in place of bottom loop segments in the target sequence. For a V_{H}(V_{H})-V_{L}(V_{L}) χ-protein construct similar to that designed in Example 2, the spliceable portions of source CDRs, flanked by the fixed top loop β-strand end points, are labelled in Figure 13 as L1'(S), L2'(S), L3(S), H1'(S) and H3'(S), while the target bottom loop segments, flanked by the fixed bottom loop β-strand end points, are labelled in Figure 13 as L1'(T) , L2'(T), L3'(T), H1'(T), H2'(T) and H3'(T). Thus, to create a χ-site L3' loop in the L BL2 loop of the target sequence, one would splice the residues identified as L3'(S) in Figure 13 to the residues flanking the segment identified as L3'(T); similarly, to create a χ-site H1' loop in the H BL4 loop of the target sequence, one would splice the residues identified as H3'(S) in Figure 13 to the residues flanking the segment identified as H1'(T), and so forth. These splices are done to create a V_{H}(V_{H})_{°}V_{L}(V_{L}) χ-protein as in Example 2. To create a V_{H}(V_{L})_{°}V_{L}(V_{H}) χ-Protein L3' loops in the H BL2 loop of the target sequence, one would splice the residues in the segment identified as L3'(S) in Figure 13 to the residues flanking the segment identified as H3'(T). Likewise, to create a χ-site H1' loop in the L BL4 loop of the target sequence, one would splice the residues flanking the region identified as H1'(S) in Figure 13 to the residues flanking the segment identified as L1'(T), and so forth.
3. Alternatively, one can build structural models for the sequences of undetermined structure using the coordinates of one or more of the known structures as a basis for the framework residue locations. Programs such as the Biosym HOMOLOGY (Biosym, Inc., San Diego, CA) program are designed to aid in this process. The procedure is well established in the literature (Grear, J. (1991) Meth. in Enzymol. 202:239-252 (1991)). Once constructed, models of the parent Fv, sFv or V region and of the Fv regions of the corresponding χ-site can thus be used to determine splice points and to construct a model of the χ-protein in the manner set forth in Examples 1 and 2. Building a model provides insight into possible steric conflicts, particularly, as was noted in Example 2, at the interface between H2' and the surface residues of the Fv domain.

Figure 14 shows the sequence of χ-protein R19.1 (D1.3) (SEQ ID NO: 42) as it would be constructed following parts 1 and 2 of the example using the aligned sequences in Figure 14. The framework and primary CDR loops are taken from R19.9 while the sequences for the χ-site loops were taken from the H1'(S), H2'(S), H3'(S), L1'(S), and L3'(S) regions of D1.3.

Figure 14 also shows the sequences of χ-protein MCP(MCP) (McPC603(McPC603) (SEQ ID NO: 41)) as it would be constructed in Examples 1 and 2. Also shown are sequences of χ-proteins 26-10(D1.3) (SEQ ID NO: 43) and 26-10(GL00P4) (SEQ ID NO: 44) as they would be constructed using the method of Example 3.

**TABLE 2**

| Light Chain Position | Residue Preference | Heavy Chain Position | Residue Preference |
|---|---|---|---|
| L1 | D,(E) | H1 | E,(D,Q) |
| L2 | I | H2 | V |
| | | H3 | Q,K,H |
| L4 | M,L | H4 | L |
| L5 | T | | |
| L6 | Q | H6 | E(Q) |
| L7 | S,T,(-) | H7 | S,(T,P,-) |
| L8 | P,(T,E) | | |
| | | H11 | L, (V) |
| | | H12 | V, (M) |
| | | H14 | P, (A) |
| L16 | G(L) | H15 | G,S |
| L17 | -,G | | |
| L20 | V, (A) | H18 | L,V,(I) |
| L21 | T(S) | H19 | K,R,S |
| L22 | I,(M,L) | H20 | L,I,M |
| L23 | S,T,(-) | H21 | S,T |
| L24 | C | H22 | C |
| L27 | S, (T) | | |
| | | H25 | S,T |
| | | H26 | G |
| | | H27 | Y,F, (D,T, -) |
| | | H28 | polar |
| | | H29 | F,I,L |
| | | H30 | T,S, (D) |
| L40 | L,M,V,(A) | | |
| L42 | W | H36 | W |
| L43 | Y,(V,I) | H37 | V,(I,M) |
| L44 | Q | H38 | K,R |
| L45 | Q, (K) | H39 | Q,(K) |
| L46 | K | | |
| L47 | P,(S) | H41 | P,(H) |
| | | H42 | G, (E) |
| | | H43 | K,Q,N,R |
| L51 | P,(V,F) | H45 | L |
| L52 | K,(R,Q) | H46 | E,(D) |
| | | H47 | W,(Y,H,D) |
| | | H48 | I,(M,L) |
| L54 | L, (W) | | |
| L55 | I,(V) | H49 | G,(A) |
| L56 | Y,(G,K) | | |
| | | H51 | I,(V,S) |
| L59 | S | | |
| | | H62 | Y,(T) |
| L64 | G | | |
| L65 | V, (I) | H66 | F, (L,V) |
| L66- | P, (S) | H67 | K, (Q,M) |
| L68 | R | H69 | K,R, (L) |
| L69 | F | | |
| L70 | S, (T) | | |
| L71 | G,(A,V) | H72 | F,I,L,V |
| L72 | S | H73 | S,T |
| L74 | S | H75 | D, (N) |
| L75 | G | | |
| L76 | T | H77 | S, (T,A,P) |
| | | H78 | polar |
| | | H79 | S,N |
| | | H80 | (-) |
| | | H81 | A,L,V |
| | | H82 | Y, (F,H) |
| L80 | L, (F) | H83 | L, (M) |
| | | H84 | Q, (D,E,K) |
| L82 | I | H85 | L,M,I |
| L83 | polar | H86 | S,N,(D,R) |
| L85 | V,M,L, (A) | H88 | L, (V) |
| L86 | Q,E | | |
| L88 | E | H91 | E, (D,A) |
| L89 | D | H92 | D |
| | | H93 | T,S |
| L91 | A,(G) | H94 | A,(G) |
| L93 | Y,(H) | H96 | Y |
| L94 | Y,F | H97 | Y,F |
| L95 | C | H98 | C |
| L96 | Q,(A,S) | H99 | A |
| | | H114 | D,A |
| L106 | T,(E,V) | | |
| L107 | F, (P) | H116 | W |
| L108 | G | H117 | G |
| | | H118 | Q,A |
| L110 | G | H119 | G |
| L111 | T | H120 | T |
| L112 | K | | |
| L113 | L, (V) | H122 | V, (L) |
| L114 | E, (T,Q,K) | H123 | T |
| L115 | I, (L,V) | H124 | V |
| L116 | K | H125 | S |
| Notes: 1) Single letter amino acid codes; letters in parentheses signify less common preferences. 2) "-" signifies the occurrence of a gap. 3) Each row corresponds to an equivalent location in the V_{H} and V_{L} chains. | | | |

### EXAMPLE 4

### Construction, Expression and Evaluation of a χ-Protein

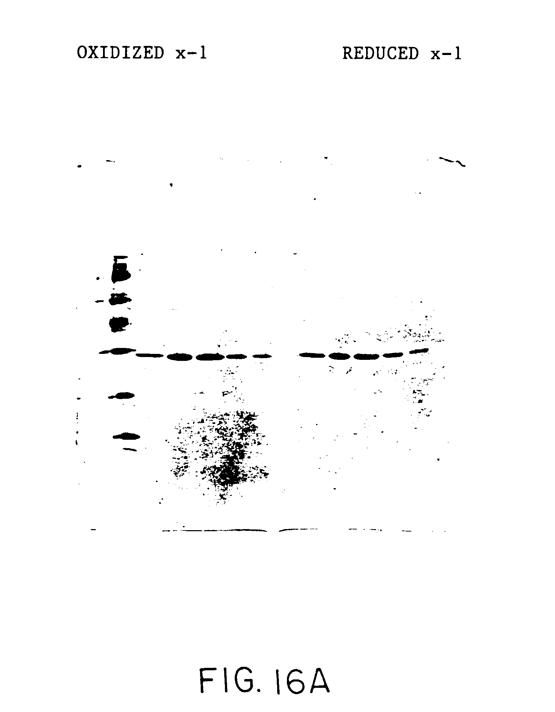

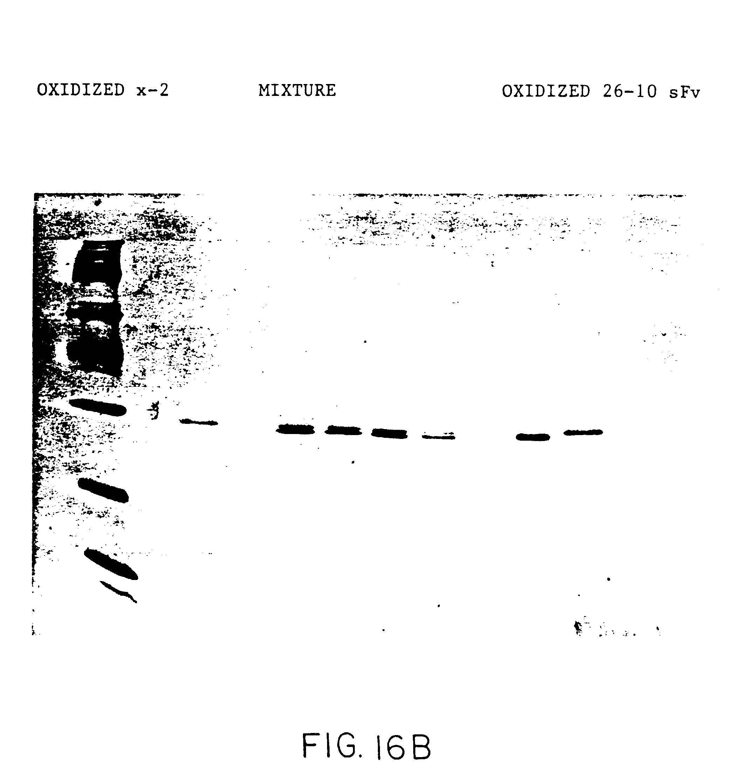

The χ-1 and χ-2 genes (Figure 15A and B, SEQ ID NOS: 45 and 47) were prepared by mutagenesis of the 26-10 sFv gene as described in (Huston, J.S., et al., Proc. Natl. Acad. Sci. USA, 85:5879-5883 (1988); Tai, M.-S., et al., Biochemistry 29:8024-8030 (1990)) and were incorporated into the pET vector described in Studier, F.W., and Moffat, B.A., J. Mol. Biol. 189:113 (1986) behind a T7 promoter. Upon transformation of E. coli with this vector, direct expression produced each in the form of cytoplasmic inclusion bodies. Cells were treated with lysozyme to allow cell lysis and ultracentrifugal isolation of the inclusion bodies, which were then dissolved in 6 M guanidinium chloride containing 10 mM dithiothreitol, 25 mM Tris, and 10 mM EDTA at pH 8.1; the solution was incubated overnight at room temperature. The protein was then diluted into 3 M or 3.5 M urea buffer containing 25 mM Tris, and 10 mM EDTA, and a glutathione redox couple (1 mM oxidized, 0.1 mM reduced) at pH 8.1. Following 18 h. at 4°C, each was fully renatured by dialysis into phosphate buffered saline (PBS), consisting of 0.05 M potassium phosphate, 0.15 M NaCl, pH 7.0, and 0.03% NaN₃. The χ-1 and χ-2 solutions were then passed through ouabainSepharose columns, washed first with PBSA (PBS +0.03% NaN₃), then with 1M NaCl in PBSA to remove any unbound material from the columns, and elution effected by displacing specifically bound protein with 20 mM ouabain in PBSA. The affinity purified χ-1 and χ-2 proteins were then examined by SDS polyacrylamide gel electrophoresis. Figure 16A and B shows the χ-1 (SEQ ID NO: 46) and χ-2 (SEQ ID NO: 48) polypeptide chains following their affinity isolation. In figure 16B, the oxidized χ-2 (upper band) is compared with 26-10 sFv (lower band) in lanes denoted as mixture. Sequence analysis of the χ-2 protein verified that the insertions noted in the gene sequence (Figure 15B) were present in the protein sequence CNBr fragments of the protein were made and sequenced as a mixture in an Applied Biosystems 470A gas-phase sequencer equipped with a model 120A on-line analyzer. The gel (Figure 16B) is also consistent with the increased molecular weight of the χ-2 over the 26-10 sFv. Refolded protein that bound to the ouabain-Sepharose column has necessarily regained active antibody combining sites for digoxin-like cardiac glycosides typified by ouabain.

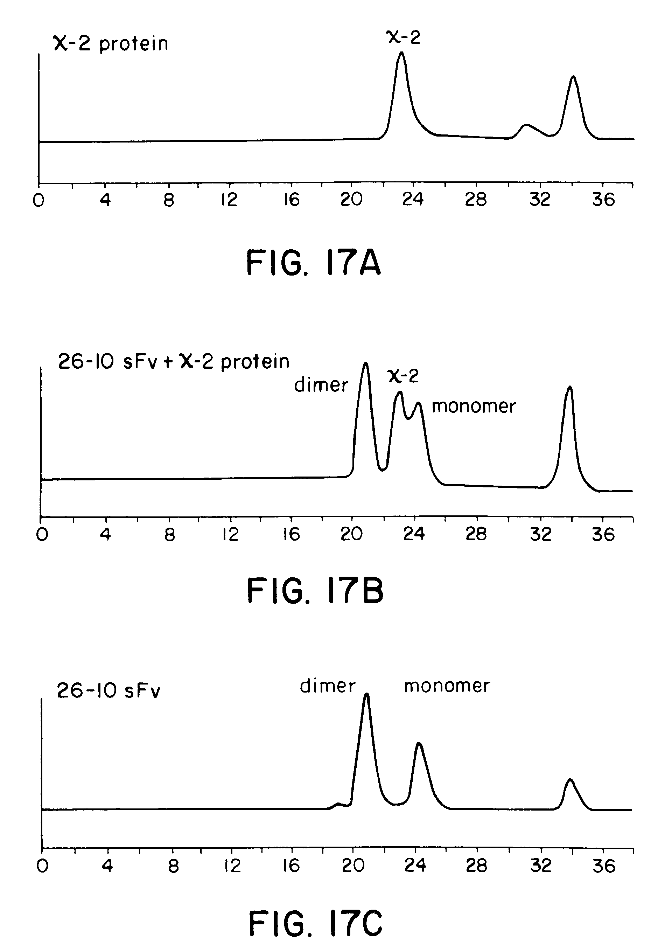

Additional insights into the shape and properties of the χ-1 and χ-2 proteins are apparent from Superdex 75 size exclusion chromatography of the affinity purified χ proteins in comparison to the 26-10 sFv, as shown in Figure 17. The single H1' insertion of χ-1 adds two tyrosyl residues within the GYGY sequence, resulting in a pronounced tendency to dimerize and a very skewed profile indicative of dissociation into monomer (data not shown). In contrast, data shown in Figure 17 for χ-2 (top panel) indicate that it appears perfectly behaved in solution, devoid of any apparent dimer. Thus, as one incorporates a multiplicity of χ-CDRs in V regions, the known hydrophobicity of CDR sequences apparently is contained by the aggregate of χ-CDR conformation and interaction. The added H1' and H2' loops necessarily increase the protein's Stokes radius, resulting in its elution position being between the 26-10 monomer and dimer positions (bottom panel). A mixing experiment that combines both proteins in a single chromatographic separation (middle panel) indicates that the χ-2 shows no apparent interaction with the 26-10 monomer and dimer species, as the middle profile appears to be a simple additive composite of the top and bottom chromatograms. The peaks beyond 30 minutes (horizontal axis is in minutes) are simply injection artifacts on the HPLC system, probably due to buffer differences between the column and sample.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A chimeric multivalent immunoglobulin (Ig) Superfamily protein analogue consisting essentially of a variable fragment (Fv) of an Ig Superfamily protein region having two ligand binding sites comprising one or more polypeptide chains forming a β-barrel domain containing complementarity-determining region-like (CDR-like) regions and framework region-like (FR-like) regions, said CDR-like regions defining a first ligand binding site and said protein analogue having a second ligand binding site segment spliced into the bottom FR-like regions of said β-barrel domain, and optionally said polypeptide chains have an amino acid sequence wherein said sequence is substituted or modified in the amino acid sequence of at least one amino acid residue.

2. A chimeric multivalent Ig Superfamily protein analogue of Claim 1 wherein (i) a non-covalently associated two chain polypeptide forms a β-barrel domain; or (ii) a single chain polypeptide forms a β-barrel domain; or (iii) comprising a single chain polypeptide forming a β-barrel domain wherein said single chain polypeptide is comprised of two polypeptide chains connected by a polypeptide linker spanning the distance between the C-terminus of one chain to the N-terminus of the other chain; or (iv) the polypeptide chain is selected from the group consisting of: heavy chain (H), light chain (L), α chain (α) β chain (β), γ chain (γ), δ chain (δ), or ε chain (ε); and optionally the protein being cross linked at sites other than ligand binding sites to form a two dimensional array of chimeric multivalent protein analogues.

3. Biological material having a nucleotide sequence which encodes a chimeric multivalent Ig Superfamily protein analogue of Claim 1, or a replicable recombinant DNA expression vector containing the nucleotide sequence.

4. A chimeric multivalent antibody analogue consisting essentially of variable fragment (Fv) region of an antibody having two ligand binding sites comprising one or more polypeptide chains forming a β-barrel domain containing complementarity determining regions (CDRs) and framework regions (FRs), said CDRs defining a first antigen binding site, said antibody analogue having a second antigen binding site segment spliced into the bottom FRs of said β-barrel domain, and optionally either:
(a) a non-covalently associated two chain polypeptide forms a β-barrel domain; or
(b) a single chain polypeptide forms a β-barrel domain; or
(c) said CDRs and FRs are comprised of heavy chain (H) polypeptide chains and light chain (L) polypeptide chains derived from variable regions (V) of immunoglobulin proteins, and optionally either (i) the CDRs may be spliced into FRs of the β-barrel domain to form an additional binding site segment such that a variable heavy chain (V_{H}) CDR is spliced into a V_{H} FR to form a V_{H}(V_{H}) polypeptide chain, or (ii) the CDRs may be spliced into FRs of the β-barrel domain to form an additional binding site segment such that a variable light chain (V_{L}) CDR is spliced into a V_{L} FR to form a V_{L}(V_{L}) polypeptide chain, or (iii) the CDRs may be spliced into FRs of the β-barrel domain to form an additional binding site segment such that a V_{H} CDR is spliced into a V_{L} FR to form a V_{L}(V_{H}) polypeptide chain, or (iv) the CDRs may be spliced into FRs of the β-barrel domain to form an additional binding site segment such that a V_{L} CDR is spliced into a V_{H} FR to form a V_{H}(V_{L}) polypeptide chain; or
(d) a single chain polypeptide forming a β-barrel domain wherein said single chain polypeptide is comprised of two polypeptide chains connected by a polypeptide linker spanning the distance between the C-terminus of one chain to the N-terminus of the other chain, and optionally said two polypeptide chains connected by a linker further comprise two V_{H}(V_{H}), V_{L}(V_{L}), V_{H}(V_{L}) or V_{L}(V_{H}) polypeptide chains, and to the N-terminal end of the polypeptide linker spanning the distance between the C-terminus of one polypeptide chain to the N-terminus of the other polypeptide chain may be added a polypeptide residue bridge which connects the N-terminal end of the linker to the C-terminal end of a CDR sequence which has been added to the C-terminal end of a FR sequence, which may comprise at least 19 amino acid residues; or
e) said CDRs and FRs are of mammalian origin, for example said CDRs and FRs may be of mouse myeloma origin.

5. Biological material having a DNA sequence which encodes the chimeric multivalent antibody analogue of Claim 4.

6. A replicable recombinant DNA expression vector containing the DNA sequence of Claim 5.

7. A chimeric multivalent Ig Superfamily protein analogue of Claim 1 wherein
(a) one binding site is reactive with a diagnostic imaging agent; or
(b) one binding site is reactive with a radiosotope; or
(c) one binding site is reactive with a cytotoxic substance; or
(d) one binding site is reactive with an effector molecule; or
(e) one binding site is reactive with a marker on a cytotoxic cell.

8. A chimeric multivalent Ig Superfamily protein analogue of Claim 1 for use in
(i) imaging specific tissue in a host comprising:
(a) administering to a host the chimeric multivalent Ig Superfamily protein analogue having one binding site reactive with a targeted tissue specific antigen and the other binding site reactive with a diagnostic imaging agent under conditions wherein said protein analogue binds to the targeted tissue; and
(b) administering the imaging agent to the host under conditions whereby said imaging agent binds to the chimeric multivalent Ig Superfamily protein analogue resulting in a detectable image of the targeted tissue; or
(ii) irradiating specific tissue in a host comprising:
(a) administering to a host the chimeric multivalent Ig Superfamily protein analogue having one binding site reactive with a targeted tissue specific antigen and the other binding site reactive with a radiosotope under conditions whereby said protein analogue binds to the targeted tissue; and
(b) administering the radioisotope to the host under conditions whereby said radioisotope binds to the chimeric multivalent Ig Superfamily protein analogue wherein binding of said chimeric protein analogue to targeted tissue and binding of said radioisotope to said chimeric protein analogue results in irradiation of the targeted tissue; or
(iii) delivering a cytotoxic substance to specific tissue in a host comprising:
(a) administering to a host the chimeric multivalent Ig Superfamily protein analogue having one binding site reactive with a targeted tissue specific antigen and the other binding site reactive with a cytotoxic substance under conditions whereby said protein analogue binds to the targeted tissue; and
(b) administering the cytotoxic substance to the host under conditions whereby said cytotoxic substance binds to the chimeric multivalent Ig Superfamily protein analogue wherein binding of said chimeric protein analogue and binding of said cytotoxic substance to said chimeric protein analogue results in delivering the toxic substance to the targeted tissue; or
(iv) lysing target cells in a host having cytotoxic cells comprising administering to a host the chimeric multivalent Ig Superfamily protein analogue having one binding site reactive with a surface receptor of a cell targeted to be lysed, and the other binding site reactive with a marker on a cytotoxic cell under conditions whereby said protein analogue binds to the targeted cell and said cytotoxic cell binds to the chimeric multivalent Ig Superfamily protein analogue, wherein binding of said chimeric protein analogue and binding of said cytotoxic cell results in lysis of the targeted cell; or
(v) modifying the function of a cell surface receptor of specific tissue in a host comprising administering to a host a chimeric multivalent Ig Superfamily protein analogue having one binding site reactive with a targeted cell surface receptor and the other binding site reactive with an effector molecule under conditions whereby said protein analogue binds to the targeted tissue and said effector molecule binds to the chimeric multivalent Ig Superfamily protein analogue, wherein binding of said chimeric protein analogue and binding of said effector molecule results in selective modification of the function of the targeted cell surface receptor.

9. The chimeric multivalent Ig Superfamily protein analogue of Claim 1 having one binding site reactive with a preselected ligand and the other binding site reactive with a substance labeled with a radioisotope or enzyme suitable for use as a quantifying agent in an in vitro diagnostic assay.

10. A method for producing a chimeric multivalent Ig Superfamily protein analogue consisting essentially of a variable fragment (Fv) of an Ig Superfamily protein region having two ligand binding sites comprising the steps of:
(a) determining the splice points for CDR-like regions to form additional ligand binding site segments on the bottom FR-like regions of a β-barrel domain whereby insertion of CDR-like region amino acid residues into the FR-like region residues maintains the folded structure required for binding activity with a preselected ligand;
(b) determining the amino acid sequence of the resulting construct having a first ligand binding site and a second ligand binding site;
(c) deducing the DNA sequence encoding the amino acid sequence of (b);
(d) synthesizing the DNA sequence;
(e) inserting the DNA sequence into an appropriate expression vector and expressing the polypeptide in a suitable host system;
(f) isolating and purifying the expressed polypeptide; and
(g) refolding the purified polypeptide to its immunologically reactive conformation, thereby resulting in a chimeric multivalent Ig Superfamily protein analogue; and optionally
wherein determining the splice points is accomplished computationally by use of a computer-generated three dimensional structure of the chimeric multivalent Ig Superfamily protein analogue, or wherein determining the splice points is accomplished by primary sequence alignment.

11. A method for effecting cell-cell interactions comprising administering to a host a chimeric multivalent Ig Superfamily protein analogue of Claim 1 having one binding site reactive with a targeted cell surface receptor of a first cell and the other binding site reactive with a targeted cell surface receptor of a second cell under conditions whereby said protein analogue binds to said first cell and second cell wherein binding of said first cell and second cell to the chimeric protein analogue results in interaction between the two cells.

12. A molecular switch comprising a chimeric multivalent Ig Superfamily protein analogue of Claim 1 having one binding site initiating a conformational change in said chimeric protein analogue when said binding site is bound to ligand, whereby the conformational change causes the chimeric protein analogue to act as a molecular switch.

13. A chimeric multivalent Ig Superfamily protein analogue according to Claim 1 for use in therapy or diagnosis, for example (a) imaging specific tissue in a host; or (b) irradiating specific tissue in a host; or (c) delivering a cytotoxic substance to specific tissue in a host; or (d) lysing target cells in a host; or (e) modifying the function of a cell surface receptor of specific tissue in a host; or (f) effecting cell-cell interactions in a host.

14. Use of a chimeric multivalent Ig Superfamily protein analogue according to Claim 1 for the manufacture of a diagnostic agent for imaging specific tissue in a host; or for the manufacture of a medicament for (a) irradiating specific tissue in a host; or (b) delivering a cytotoxic substance to specific tissue in a host; or (c) lysing target cells in a host; or (d) modifying the function of a cell surface receptor of specific tissue in a host; or (e) effecting cell-cell interactions in a host.

## Patentansprüche

1. Ein chimäres mehrwertiges Protein-Analogon der Immunoglobulin (Ig) Superfamilie, das im wesenlichen aus einem variablen Fragment (Fv) einer Proteinregion der Ig Superfamilie besteht, zwei Ligandbindungsstellen besitzt, eine oder mehrere Peptidketten umfaßt, die eine β-Faß Domäne bilden, die komplementaritätsbeatimmende Region-ähnliche (CDR-ähnliche) Regionen und Gerüstregion-ähnliche (FR-ähnliche) Regionen enthält, wobei besagte CDR-ähnliche Regionen eine erste Ligandbindungsstelle definieren und besagtes Protein-Analogon ein in die FR-ähnlichen Boden-Regionen von besagter β-Raß Domäne gespleißtes zweites Ligandbindungestellensegment besitzt, und gegebenenfalls besagte Polypeptidketten eine Aminosäure-Sequenz besitzen, worin besagte Sequenz in der Aminosäure-Sequenz mindestens in einem Aminosäure-Rest substituiert oder modifiziert ist.

2. Ein chimäres mehrwertiges Protein-Analogon der Ig Superfamilie nach Anspruch 1, worin (i) ein nicht-kovalent assoziiertes zweikettiges Polypeptid eine β-Faß Domäne bildet; oder (ii) ein einzelkettiges Polypeptid eine β-Faß Domäne bildet; oder (iii) umfassend ein einzelkettiges Polypeptid, das eine β-Faß Domäne bildet, worin besagtes einzelkettiges Polypeptid aus zwei Polypeptidketten besteht, die über einen Polypeptid-Linker verbunden sind, der sich über die Distanz zwischen dem C-Terminus von einer Kette bis zum N-Terminus der anderen Kette erstreckt; oder (iv) die Polypeptidkette aus der Gruppe gewählt ist bestehend aus: schwerer Kette (H), leichter Kette (L), α Kette (α) β Kette (β), γ Kette (γ), δ Kette (δ) oder ε Kette (ε) ; und wobei gegebenenfalls das Protein an anderen Stellen als Ligandbindungsstellen vernetzt ist, um einen zweidimensionalen Bereich von chimären mehrwertigen Protein-Analoga zu bilden.

3. Biologisches Material mit einer Nucleotid-Sequenz, die ein chimäres mehrwertiges Protein-Analogon der Ig Superfamilie nach Anspruch 1 codiert, oder ein replizierbarer rekombinanter DNS-Expressionavektor, der die NucLeotid-Sequenz enthält.

4. Ein chimäres mehrwertiges Antikörper-Analogon, das im wesentlichen aus einer variablen Fragment (Fv) Region eines Antikörpers besteht, zwei Ligandbindungsstellen besitzt, eine oder mehrere Polypeptidketten umfaßt, die eine β-Faß Domäne bilden, die komplementaritätsbestimmende Regionen (CDRs) und Gerüstregionen (FRs) enthalten, wobei besagte CDRs eine erste Antigenbindungsstelle definieren, wobei besagtes AntikörperAnalogon ein in die Boden-FRs von besagter β-Faß Domäne gespleißtes zweites Antigenbindungsstellensegment besitzt, und gegebenenfalls entweder:
(a) ein nicht-kovalent assoziiertes zweikettiges Polypeptid eine β-Faß Domäne bildet; oder
(b) ein einzelkettiges Polypeptid eine β-Faß Domäne bildet; oder
(c) besagte CDRs und FRs schwerkettige (H) Polypeptidketten und leichtkettige (L) Polypeptidketten umfassen, die von variablen Regionen (V) von Immunoglobulin-Proteinen abstammen, und gegebenenfalls entweder (i) können die CDRs in FRs der β-Faß Domäne gespleißt sein, um ein zusätzliches Bindungsstellensegment zu bilden, so daß eine variable schwere Kette (V_{H}) CDR in eine V_{H} FR gespleißt ist, um eine eine V_{H} (V_{H}) Polypeptidkette zu bilden, oder (ii) können die CDRs in FRs der β-Faß Domäne gespleißt sein, um ein zusätzliches Bindungsstellensegment zu bilden, so daß eine variable leichte Kette (V_{H}) CDR in eine V_{L} FR gespleißt ist, um eine V_{L} (V_{L}) Polypeptidkette zu bilden, oder (iii) können die CDRs in FRs der β-Faß Domäne gespleißt sein, um ein zusätzliches Bindungsstellensegment zu bilden, so daß eine V_{H} CDR in eine V_{L} FR gespleißt ist, um eine V_{L} (V_{H}) Polypeptidkette zu bilden, oder (iv) die CDRs können in FRs der β-Faß Domäne gespleißt sein, um ein zusätzliches Bindungsstellensegment zu bilden, so daß eine V_{L} CDR in eine V_{H} FR gespleißt ist, um eine V_{H} (V_{L}) Polypeptidkette zu bilden; oder
(d) ein einzelkettiges Polypeptid, das eine β-Faß Domäne bildet, worin besagtes einzelkettiges Polypeptid zwei Polypeptidketten umfaßt, die über einen Polypeptid-Linker verbunden sind, der sich über die Distanz zwischen dem C-Terminus von einer Kette zum N-Terminus der anderen Kette erstreckt, und gegebenenfalls besagte über einen Linker verbundene zwei Polypeptidketten umfassen ferner zwei V_{H} (V_{H}), V_{L}(V_{L}), V_{H}(V_{L}) oder V_{L}(V_{H}) Polypeptidketten, und an dem N-terminalen Ende des Polypeptid-Linkers, der sich über die Distanz zwischen dem C-Terminus von einer Polypeptidkette zum N-Terminus der anderen Polypeptidkette erstreckt, kann eine Polypeptidrest-Brücke angefügt sein, die das N-terminale Ende des Linkers mit dem C-terminalen Ende einer CDR-Sequenz verbindet, die an das C-terminale Ende einer FR-Sequenz angefügt worden ist, die mindestens 19 Aminosäure-Reste umfassen kann; oder
(e) besagte CDRs und FRs sind Säuger-Ursprungs, zum Beispiel können besagte CDRs und FRs Maus Myeloma Ursprungs sein.

5. Biologisches Material mit einer Nucleotid-Sequenz, die ein chimäres mehrwertiges Antikörper-Analogon nach Anspruch 4 codiert.

6. Ein replizierbarer rekombinanter DNS-Expressionsvektor, der die DNS-Sequenz nach Anspruch 5 enthält.

7. Ein chimäres mehrwertiges Protein-Analogon der Ig Superfamilie nach Anspruch 1, worin
(a) eine Bindungsstelle mit einem diagnostischen bilderzeugenden Agens reaktiv ist; oder
(b) eine Bindungsstelle mit einem Radioisotop reaktiv ist; oder
(c) eine Bindungsstelle mit einer cytotoxischen Substanz reaktiv ist; oder
(d) eine Bindungsstelle mit einem Effektor-Molekül reaktiv ist; oder
(e) eine Bindungsstelle mit einem Marker auf einer cytotoxischen Zelle reaktiv ist.

8. Ein chimäres mehrwertiges Protein-Analogon der Ig Superfamilie nach Anspruch 1 zur Verwendung für
(i) eine bildliche Darstellung von spezifischem Gewebe in einem Wirt, die umfaßt:
(a) Verabreichen des chimären mehrwertigen Protein-Analogons der Ig Superfamilie mit einer Bindungsstelle, die mit einem Zielgewebe-spezifischen Antigen reaktiv ist, und der anderen Bindungsstelle, die mit einem diagnostischen bilderzeugenden Agens reaktiv ist, an einen Wirt unter Bedingungen, worin besagtes Protein-Analogon an das Zielgewebe bindet; und
(b) Verabreichen des bilderzeugenden Agens an den Wirt unter Bedingungen, wodurch besagtes bilderzeugende Agens an das chimäre mehrwertige Protein-Analogon der Ig Superfamilie bindet, was zu einem detektierbaren Bild des Zielgewebes führt; oder
(ii) Bestrahlen von spezifischem Gewebe in einem Wirt, das umfaßt:
(a) Verabreichen des chimären mehrwertigen Protein-Analogone der Ig Superfamilie mit einer Bindungsstelle, die mit einem Zielgewebe-spezifischen Antigen reaktiv ist, und der anderen Bindungsstelle, die mit einem Radioisotop reaktiv ist, an einen Wirt unter Bedingungen, wodurch besagtes Protein-Analogon an das zielgewebe bindet; und
(b) Verabreichen des Radioisotops an den Wirt unter Bedingungen, wodurch besagtes Radioisotop an das chimäre mehrwertige Protein-Analogon der Ig Superfamilie bindet, worin ein Binden besagten chimären Protein-Analogons an das Zielgewebe und ein Binden besagten radioisotops an besagtes chimäres Protein-Analogon zur Bestrahlung des Zielgewebes führt; oder
(iii) Überbringen einer cytotoxischen Substanz an ein spezifisches Gewebe in einem Wirt, das umfaßt:
(a) Verabreichen des chimären mehrwertigen Protein-Analogons der Ig Superfamilie mit einer Bindungsstelle, die mit einem Zielgewebespezifischen Antigen reaktiv ist, und der anderen Bindungsstelle, die mit einer cytotoxischen Substanz reaktiv ist, an einen Wirt unter Bedingungen, wodurch besagtes Protein-Analogon an das Zielgewebe bindet; und
(b) Verabreichen der cytotoxischen Substanz an den Wirt unter Bedingungen, wodurch besagte cytotoxische Substanz an das chimäre mehrwertige Protein-Analogon der Ig Superfamilie bindet, worin ein Binden von besagtem Protein-Analogon und ein Binden von besagter cytotoxischer Substanz an besagtes chimäres Protein-Analogon zu einem Überbringen der toxischen Substanz an das Zielgewebe führt; oder
(iv) Lysieren von Zielzellen in einem Wirt mit cytotoxischen Zellen, das Verabreichen des chimären mehrwertigen Protein-Analogons der Ig Superfamilie mit einer Bindungsstelle, die mit einem Oberflächenrezeptor einer zu lysierenden Zielzelle reaktiv ist, und der anderen Bindungsstelle, die mit einem Marker auf einer cytotoxischen Zelle reaktiv ist, an einen Wirt unter Bedingungen umfaßt, wodurch besagtes Protein-Analogon an die Ziellzelle bindet und besagte cytotoxische Zelle an das chimäre mehrwertige Protein-Analogon der Ig Superfamilie bindet, worin ein Binden von besagtem chimären Protein-Analogon und ein Binden von besagter cytotoxischer Zelle zu einer Lyse der Zielzelle führt; oder
(v) Modifizieren der Funktion eines Zelloberflächenrezeptors eines spezifischen Gewebes in einem Wirt, wobei das Modifizieren ein Verabreichen eines chimären mehrwertigen Protein-Analogons der Ig Superfamilie mit einer Bindungsstelle, die mit einem Zielzelloberflächenrezeptor reaktiv ist, und der anderen Bindungsstelle, die mit einem Effektormolekül reaktiv ist, an einen Wirt unter Bedingungen umfaßt, wodurch besagtes Protein-Analogon an das Zielgewebe bindet und besagtes Effektormolekül an das chimäre mehrwertige protein-Analogon der Ig Superfamilie bindet, worin ein Binden von besagtem chimären Protein-Analogon und ein Binden von besagtem Effektormolekül zu einer selektiven Modifikation der Funktion des Zielzelloberflächenrezeptors führt.

9. Das chimäre mehrwertige Protein-Analogon der Ig Superfamilie nach Anspruch 1 mit einer Bindungsstelle, die mit einem vorgewählten Liganden reaktiv ist, und der anderen Bindungsstelle, die mit einer Substanz reaktiv ist, die mit einem zur Verwendung als ein Mittel zur quantitativen Bestimmung in einem in vitro diagnostischen Test geeigneten Radioisotop oder Enzym markiert ist.

10. Ein Verfahren zur Herstellung eines chimären mehrwertigen Protein-Analogons der Ig Superfamilie, das im wesentlichen aus einem variablen Fragment (Fv) einer Proteinregion der Ig Superfamilie besteht und zwei Ligandbindungsstellen besitzt, wobei das Verfahren die Schritte umfaßt:
(a) Bestimmen der Spleiß-Punkte für CDR-ähnliche Regionen, um zusätzliche Ligandbindungsstellensegmente am unteren Ende der FR-ähnlichen Regionen einer β-Faß Domäne zu bilden, wodurch eine Insertion von CDR-ähnliche Region-Aminosäure-Resten in die FR-ähnliche Region-Reste die gefaltete Struktur aufrecht erhält, die für eine Bindungsaktivität mit einem vorgewählten Liganden erforderlich ist;
(b) Bestimmen der Aminosäure-Sequenz des resultierenden Konstrukts, das eine erste Ligandbindungsstelle und eine zweite Ligandbindungsstelle besitzt;
(c) Herleiten der DNS-Sequenz, die die Aminosäure-Sequenz von (b) codiert;
(d) Synthetisieren der DNS-Sequenz;
(e) Inserieren der DNS-Sequenz in einen geeigneten Expressionsvektor und Expression des Polypeptids in einem geeigneten Wirtssystem;
(f) Isolieren und Reinigen des exprimierten Polypeptids; und
(g) Wiederfalten des gereinigten Polypeptids in seine immunologisch reaktive Konformation, dadurch wird ein chimäres mehrwertiges Protein-Analogon der Ig Superfamilie erhalten; und gegebenenfalls
worin eine bestimmung der Spleiß-Punkte durch Verwendung einer Computer-generierten dreidimensionalen Struktur des chimären mehrwertigen Protein-Analogons der Ig Superfamilie rechnerisch ausgeführt ist ader worin eine bestimmung der Spleiß-Punkte durch ein Alignment der Primärsequenz ausgeführt ist.

11. Ein Verfahren zur Beeinflussung von Zell-Zellwechselwirkungen, das Verabreichen des chimären mehrwertigen Protein-Analogons der Ig Superfamilie nach Anspruch 1 mit einer Bindungsstelle, die mit einem Zielzelloberflächenrezeptor einer ersten Zelle reaktiv ist, und der anderen Bindungsstelle, die mit einem Zielzelloberflächenrezeptor einer zweiten Zelle reaktiv ist, an einen Wirt unter Bedingungen umfaßt, wodurch besagtes Protein-Analogon an besagte erste Zelle und zweite Zelle bindet, worin ein Binden von besagter erster Zelle und zweiter Zelle an das chimäre Protein-Analogon zu einer Wechselwirkung zwischen den beiden Zellen führt.

12. Ein molekularer Schalter, der ein chimäres mehrwertiges Protein-Analogon der Ig Superfamilie nach Anspruch 1 mit einer Bindungsstelle umfaßt und eine Konformationsänderung in besagtem chimären Protein-Analogon initiiert, wenn besagte Bindungsstelle an einen Liganden gebunden ist, wodurch die Konformationsänderung das chimäre Protein-Analogon als einen molekularen Schalter fungieren läßt.

13. Ein chimäres mehrwertiges Protein-Analogon der Ig Superfamilie gemäß Anspruch 1 zur Verwendung in Therapie oder Diagnose, zum Beispiel (a) Bildlichen Darstellung von spezifischem Gewebe in einem Wirt; oder (b) Bestrahlen von spezifischem Gewebe in einem Wirt; oder (c) Überbringen einer cytotoxischen Substanz an spezifisches Gewebe in einem Wirt; oder (d) Lysieren von Zielzellen in einem Wirt; oder (e) Modifizieren der Funktion eines Zelloberflächenrezeptors von spezifischem Gewebe in einem Wirt; oder (f) Beeinflussen von Zell-Zellwechselwirkungen in einem Wirt.

14. Verwendung eines chimären mehrwertigen Protein-Analogons der Ig Superfamilie gemäß Anspruch 1 zur Herstellung eines diagnostischen Mittels zur bildlichen Darstellung von spezifischem Gewebe in einem Wirt; oder zur Herstellung eines Medikaments zum (a) Bestrahlen von spezifischem Gewebe in einem Wirt; oder (b) Überbringen einer cytotoxischen Substanz an spezifisches Gewebe in einem Wirt; oder (c) Lysieren von Zielzellen in einem Wirt; oder (d) Modifizieren der Funktion eines Zelloberflächenrezeptors von spezifischem Gewebe in einem Wirt; oder (e) Beeinflussen von Zell-Zellwechselwirkungen in einem Wirt.

## Revendications

1. Analogue protéique chimérique multivalent de la Superfamille des immunoglobulines (Ig) consistant essentiellement en un fragment variable (Fv) d'une région d'une protéine de la Superfamille des Ig ayant deux sites de liaison à un ligand comprenant une ou plusieurs chaînes polypeptidiques formant un domaine formant un tonneau β contenant des régions similaires à la région de détermination de la complémentarité (CDR-like) et des régions similaires à des régions de squelette (FR-like), lesdites régions CDR-like définissant un premier site de liaison à un ligand et ledit analogue protéique ayant un second segment formant un site de liaison à un ligand épissé à l'inférieur des régions FR-like au fond dudit domaine formant un tonneau β, et éventuellement lesdites chaînes polypeptidiques ayant une séquence d'acides aminés substituée ou modifiée sur au moins un résidu d'acides aminés.

2. Analogue protéique chimérique multivalent de la Superfamille des Ig selon la revendication 1, dans lequel (i) un polypeptide à deux chaînes associées de manière non-covalente forme un domaine en tonneau β; ou (ii) un polypeptide à simple chaîne forme un domaine en tonneau β; ou (iii) comprenant un polypeptide à simple chaîne formant un domaine en tonneau β dans lequel ledit polypeptide à simple chaîne est composé de deux chaînes polypeptidiques liées par un polypeptide linker augmentant la distance entre l'extrémité C-terminale d'une chaîne et l'extrémité N-terminale de l'autre chaîne ; ou (iv) la chaîne polypeptidique est choisie parmi le groupe consistant en une chaîne lourde (H), une chaîne légère (L), une chaîne α (α), une chaîne β (β), une chaîne γ (γ), une chaîne δ (δ) ou une chaîne ε (ε) ; et éventuellement la protéine étant liée de manière croisée à des sites autres que les sites de liaison au ligand pour former un réseau à deux dimensions d'analogues protéiques chimériques multivalents.

3. Matériel biologique ayant une séquence nucléotidique qui code pour un analogue protéique multivalent chimérique de la Superfamille des Ig selon la revendication 1, ou un vecteur d'expression d'ADN recombinant réplicable contenant la séquence nucléotidique.

4. Analogue d'anticorps multivalent chimérique consistant essentiellement en une région de fragment variable (Fv) d'un anticorps ayant deux sites de liaison à un ligand comprenant une ou plusieurs chaînes polypeptidiques formant un domaine formant un tonneau β contenant des régions de détermination de la complémentarité (CDR) et des régions de squelette (FR), lesdites CDR définissant un premier site de liaison à l'antigène, ledit analogue de l'anticorps ayant un second segment formant un site de liaison à l'antigène épissé à l'intérieur des régions FR au fond dudit domaine formant un tonneau β, analogue dans lequel éventuellement soit :
(a) un polypeptide à deux chaînes associées de manière non covalente forme un domaine formant un tonneau β; ou
(b) un polypeptide à simple chaîne forme un domaine formant un tonneau β ; ou
(c) lesdites régions CDR et FR sont composées des chaînes polypeptidiques de chaînes lourdes (H) et des chaînes polypeptidiques de chaînes légères (L) dérivées des régions variables (V) des protéines d'immunoglobulines, et éventuellement soit (i) les CDR peuvent être épissées à l'intérieur des régions FR du domaine formant un tonneau β pour former un segment formant un site de liaison supplémentaire de sorte qu'une région CDR de la chaîne lourde variable (V_{H}) soit épissée à l'intérieur FR de V_{H} pour former une chaîne polypeptidique V_{H}(V_{H}), ou (ii) les régions CDR peuvent être épissées à l'intérieur des régions FR du domaine formant un tonneau β pour former un site de liaison supplémentaire de sorte qu'une région CDR d'une chaîne légère variable (V_{L}) est épissée à l'intérieur d'une région FR de V_{L} pour former une chaîne polypeptidique V_{L}(V_{L}), ou (iii) les régions CDR peuvent être épissées à l'intérieur des régions FR du domaine formant un tonneau β pour former un segment formant un site de liaison supplémentaire tel qu'une région CDR de V_{H} soit épissée à l'intérieur d'une région FR de V_{L} pour former une chaîne polypeptidique V_{L}(V_{H}); ou (iv) les régions CDR peuvent être épissées à l'intérieur des régions FR du domaine formant un tonneau β pour former un segment formant un site de liaison supplémentaire de sorte qu'une région CDR de V_{L} soit épissée à l'intérieur d'une région FR de V_{H} pour former une chaîne polypeptidique V_{H}(V_{L}) ; ou
(d) un polypeptide à simple chaîne forme un domaine formant un tonneau β, ledit polypeptide à simple chaîne étant composé de deux chaînes polypeptidiques liées par un polypeptide linker augmentant la distance entre l'extrémité C-terminale d'une chaîne et l'extrémité N-terminale de l'autre chaîne, et éventuellement lesdites chaînes polypeptidiques liées par un linker comprennent en outre deux chaînes polypeptidiques V_{H}(V_{H}), V_{L}(V_{L}), V_{H}(V_{L}) ou V_{L}(V_{H}), et, à l'extrémité N-terminale du polypeptide linker augmentant la distance entre l'extrémité C-terminale d'une chaîne polypeptidique et l'extrémité N-terminale de l'autre chaîne polypeptidique peut être ajouté un pont formé de résidus polypeptidiques qui lie l'extrémité N-terminale du linker à l'extrémité C-terminale d'une séquence CDR qui a été ajoutée à l'extrémité C-terminale d'une séquence FR, qui peut comprendre au moins 19 résidus d'acides aminés ; ou
(e) lesdites régions CDR et FR ont une origine mammifère, par exemple lesdites régions CDR et FR peuvent avoir comme origine un myélome de souris.

5. Matériel biologique ayant une séquence d'ADN qui code pour un analogue d'anticorps chimérique multivalent selon la revendication 4,

6. Vecteur d'expression d'ADN recombinant réplicable contenant la séquence d'ADN selon la revendication 5

7. Analogue protéique chimérique multivalent de la Superfamille des Ig selon la revendication 1 dans lequel
(a) un site de liaison est capable de réagir avec un agent d'imagerie diagnostique; ou
(b) un site de liaison est capable de réagir avec un radioisotope ; ou
(c) un site de liaison est capable de réagir avec une substance cytotoxique ; ou
(d) un site de liaison est capable de réagir avec une molécule effectrice; ou
(e) un site de liaison est capable de réagir avec un marqueur d'une cellule cytotoxique.

8. Analogue protéique chimérique multivalent de la Superfamille des Ig selon la revendication 1 pour l'utilisation dans
(i) l'imagerie d'un tissu spécifique chez un hôte comprenant
(a) l'administration à un hôte d'un analogue protéique chimérique multivalent de la Superfamille des Ig ayant un site de liaison pouvant réagir avec un antigène spécifique du tissu ciblé et l'autre site de liaison pouvant réagir avec un agent d'imagerie diagnostique dans des conditions selon lesquelles ledit analogue protéique se lie audit tissu cible ; et
(b) l'administration de l'agent d'imagerie à l'hôte dans des conditions selon lesquelles ledit agent d'imagerie se lie à l'analogue protéique chimérique multivalent de la Superfamille des lg, résultant en une image détectable du tissu ciblé ; ou
(ii) l'irradiation d'un tissu spécifique chez un hôte comprenant :
(a) l'administration à un hôte d'un analogue protéique chimérique multivalent de la Superfamille des lg ayant un site de liaison pouvant réagir avec un antigène spécifique du tissu ciblé et l'autre site de liaison pouvant réagir avec un radioisotope dans des conditions selon lesquelles ledit analogue protéique se lie audit tissu cible ; et
(b) l'administration du radioisotope à l'hôte dans des conditions selon lesquelles ledit radioisotope se lie à l'analogue protéique chimérique multivalent de la Superfamille des Ig, la liaison dudit analogue protéique chimérique au tissu ciblé et la liaison dudit radioisotope audit analogue protéique chimérique, résultant dans l'irradiation du tissu ciblé ; ou
(iii) la libération d'une substance cytotoxique à un tissu spécifique chez un hôte comprenant :
(a) l'administration à un hôte d'un analogue protéique chimérique multivalent de la Superfamille des lg ayant un site de liaison pouvant réagir avec un antigène spécifique du tissu ciblé et l'autre site de liaison pouvant réagir avec une substance cytotoxique dans des conditions selon lesquelles ledit analogue protéique se lie audit tissu cible ; et
(b) l'administration de la substance cytotoxique à l'hôte dans des conditions selon lesquelles ladite substance cytotoxique se lie à l'analogue protéique chimérique multivalent de la Superfamille des Ig, la liaison de ladite substance cytotoxique à ladite protéine chimérique au tissu ciblé et la liaison de ladite substance cytotoxique audit analogue protéique chimérique, résultant dans la libération de la substance cytotoxique au tissu ciblé ; ou
(iv) la lyse des cellules cibles chez un hôte ayant des cellules cylotoxiques comprenant l'administration à un hôte de l'analogue protéique chimérique multivalent de la Superfamille des lg ayant un site de liaison capable de réagir avec un récepteur de surface d'une cellule cible à lyser, et l'autre site de liaison capable de réagir avec un marqueur sur une cellule cytotoxique dans des conditions selon lesquelles ledit analogue protéique se lie à la cellule cible et ladite cellule cytotoxique se lie audit analogue protéique chimérique multivalent de la Superfamille des Ig, la liaison dudit analogue protéique chimérique et la liaison de ladite cellule cytotoxique résultant dans la lyse de la cellule ciblée ; ou
(v) la modification de la fonction d'un récepteur de surface cellulaire d'un tissu spécifique chez un hôte comprenant l'administration à un hôte d'un analogue protéique chimérique multivalent de la Superfamille des Ig ayant un site de liaison capable de réagir avec un récepteur de surface cellulaire cible et l'autre site de liaison capable de réagir avec une molécule effectrice dans des conditions selon lesquelles ledit analogue protéique se lie audit tissu cible et ladite molécule effectrice se lie à l'analogue protéique chimérique multivalent de la Superfamille des Ig, la liaison dudit analogue protéique chimérique et la liaison de ladite molécule effectrice résultant dans la modification sélective de la fonction du récepteur de surface cellulaire cible.

9. Analogue protéique chimérique multivalent de la Superfamille des Ig selon la revendication 1 ayant un site de liaison capable de réagir avec un ligand présélectionné et l'autre site de liaison capable de réagir avec une substance marquée par un radioisotope ou une enzyme appropriée pour une utilisation en tant qu'agent de quantification dans un test de diagnostic *in vitro*.

10. Procédé de production d'un analogue protéique chimérique multivalent de la Superfamille des Ig consistant essentiellement en un fragment variable (FV) d'une région d'une protéine de la Superfamille des Ig ayant deux sites de liaison à un ligand, comprenant les étapes consistant à :
(a) déterminer les points d'épissage des régions CDR-like pour former les segments formant le site de liaison supplémentaire au ligand à l'intérieur des régions FR-like au fond d'un domaine formant un tonneau β, l'insertion de résidus d'acides aminés de la région CDR-like à l'intérieur des résidus de la région FR-like maintenant la structure repliée requise pour l'activité de liaison avec un ligand présélectionné ;
(b) déterminer la séquence d'acides aminés de la construction résultante ayant un premier site de liaison au ligand et un second site de liaison au ligand ;
(c) déduire la séquence d'ADN codant pour la séquence d'acides aminés de (b);
(d) synthétiser la séquence d'ADN ;
(e) insérer la séquence d'ADN dans un vecteur d'expression approprié et exprimer le polypeptide dans un système hôte approprié ;
(f) isoler et purifier le polypeptide exprimé; et
(g) effectuer le repliement du polypeptide purifié dans sa conformation immunologiquement réactive, résultant en un analogue protéique chimérique multivalent de la Superfamille des Ig ; et éventuellement
dans lequel la détermination des points d'épissage est accomplie par ordinateur par utilisation d'une structure tridimensionnelle générée par ordinateur d'un analogue protéique chimérique multivalent de la Superfamille des lg, ou dans lequel la détermination des points d'épissage est accomplie par l'alignement des séquences primaires.

11. Procédé pour réaliser les interactions cellule-cellule comprenant l'administration à un hôte d'un analogue protéique chimérique multivalent de la Superfamille des Ig selon la revendication 1 ayant un site de liaison capable de réagir avec un récepteur de surface cellulaire cible d'une première cellule et l'autre site de liaison capable de réagir avec un récepteur de surface cellulaire cible d'une seconde cellule dans des conditions selon lesquelles ledit analogue protéique se lie à ladite première cellule et à ladite seconde cellule, la liaison de ladite première cellule et la seconde cellule à l'analogue protéique chimérique résultant dans l'interaction entre les deux cellules.

12. Commutateur moléculaire comprenant un analogue protéique chimérique multivalent de la Superfamille des Ig selon la revendication 1 ayant un site de liaison initiant un changement conformationnel dans ledit analogue protéique chimérique lorsque ledit site de liaison est lié au ligand, le changement conformationnel permettant à l'analogue protéique chimérique de réagir comme commutateur moléculaires

13. Analogue protéique chimérique multivalent de la Superfamille des Ig selon la revendication 1 pour une utilisation en thérapie ou en diagnostic, par exemple (a) l'imagerie d'un tissu spécifique chez un hôte ; où (b) l'irradiation d'un tissu spécifique chez un hôte ; (c) la libération d'une substance cytotoxique à un tissu spécifique chez un hôte ; (d) la lyse de cellules cibles chez un hôte ; (e) la modification de la fonction d'un récepteur de surface cellulaire d'un tissu spécifique chez un hôte ; ou (f) la réalisation d'interactions cellule-cellule chez un hôte.

14. Utilisation d'un analogue protéique chimérique multivalent de la Superfamille des Ig selon la revendication 1 pour la fabrication d'un agent de diagnostic pour l'imagerie d'un tissu spécifique chez un hôte ; ou pour la fabrication d'un médicament pour (a) l'irradiation d'un tissu spécifique chez un hôte ou (b) l'irradiation d'un tissu spécifique chez un hôte ; (c) la libération d'une substance cytotoxique à un tissu spécifique chez un hôte ; (d) la lyse de cellules cibles chez un hôte; (e) la modification de la fonction d'un récepteur de surface cellulaire d'un tissu spécifique chez un hôte; ou (f) la réalisation d'interactions cellule-cellule chez un hôte.
